# EUROPEAN PATENT SPECIFICATION

(11) **EP 3 187 514 B1**
(45) Date of publication and mention of the grant of the patent: **29.07.2020**
(21) Application number: 15835738.4
(22) Date of filing: 25.08.2015
(51) Int. Cl.: C07C 69/76, C07C 69/83, C08F 20/30, C07C 69/94, C07C 235/12, C07C 317/44, C07C 323/62, C07C 271/16

(54) **POLYMERIZABLE MONOMER, CURABLE COMPOSITION AND RESIN MEMBER**
POLYMERISIERBARES MONOMER, HÄRTBARE ZUSAMMENSETZUNG UND HARZELEMENT
MONOMÈRE POLYMÉRISABLE, COMPOSITION DURCISSABLE ET ARTICLE EN RÉSINE

(30) Priority: 26.08.2014 JP 2014171513
(43) Date of publication of application: 05.07.2017
(73) Proprietor: Tokuyama Dental Corporation, Tokyo 110-0016 (JP)
(72) Inventor: INAKI, Yoshitaka, Tokyo 110-0016 (JP); SAKATA, Eibu, Tokyo 110-0016 (JP); SUZUKI, Takeshi, Tokyo 110-0016 (JP)
(74) Representative: Gill Jennings & Every LLP
(86) International application number: PCT/JP2015/073910
(87) International publication number: WO 2016/031831

(56) References cited:
- EP-A1- 2 985 639
- EP-A2- 0 107 621
- EP-A2- 0 388 979
- WO-A1-85/00514
- WO-A2-2011/139936
- JP-A- H03 134 005
- JP-A- S62 277 412
- JP-A- 2012 025 959
- KR-A- 20140 137 802
- WEN-YEN CHIANG ET AL: "Synthesis and properties of ultraviolet-curable resins via a thio-ene (thiol and allyl) addition reaction", JOURNAL OF APPLIED POLYMER SCIENCE, WILEY, vol. 86, 1 January 2002 (2002-01-01), pages 1878-1885, XP002534890, ISSN: 0021-8995, DOI: 10.1002/APP.11117

## Description

### Technical Field

The present invention relates to a polymerizable monomer, a method of producing the polymerisable monomer, a curable composition, and a resin member.

### Background Art

A (meth)acrylate-based polymerizable monomer may be utilized in a wide range of fields, such as a dental material like a dental curable composition or a dental adhesive, an optical material, a printing plate, a photoresist material, a paint, an adhesive, an ink, and a stereolithographic resin (for example, Patent Literatures 1 to 4). In particular, as a (meth)acrylate-based polymerizable monomer from which a cured product excellent in mechanical strength is obtained, there are known a polymerizable monomer having a bisphenol A skeleton typified by bisphenol A diglycidyl di(meth)acrylate (Bis-GMA) exemplified in Patent Literatures 2 and 3, and a polymerizable monomer having a biphenyl skeleton exemplified in Patent Literatures 4 and 5.

### Citation List

### Patent Literature

[PTL 1] JP 2008-534256 A
[PTL 2] JP 2007-126417 A
[PTL 3] JP 09-157124 A
[PTL 4] JP 05-170705 A
[PTL 5] JP 63-297344 A

### Summary of Invention

### Technical Problem

Meanwhile, from the viewpoint of facilitating the handling property of a polymerizable monomer, it is advantageous that the polymerizable monomer is a liquid or liquid-like substance having low viscosity under a room temperature environment. For example, in general, in many cases, the polymerizable monomer is used as a mixed composition having mixed therein other components depending on various use applications, rather than being used alone. In such cases, when the polymerizable monomer is a liquid or liquid-like substance having low viscosity, its blending with other components is extremely easy.

However, Bis-GMA exemplified in Patent Literatures 2 and 3 has extremely high viscosity under a room temperature environment, and the polymerizable monomer exemplified in Patent Literature 4 is even a solid under a room temperature environment, and besides, is poor in mechanical strength. In addition, the polymerizable monomer exemplified in Patent Literature 5 is also poor in mechanical strength, though having relatively low viscosity. Further, WO2011/139936 discloses a self-etching and self-adhesive dental restorative composition including a polymerizable compound having at least one phosphorus containing acidic group, a polymerizable compound having at least one carboxylic acid group, and a copolymerizable multi-functional (meth)acrylate monomer.

The present invention has been made in view of the above-mentioned circumstances, and an object of the present invention is to provide a polymerizable monomer excellent in mechanical strength of its cured product and excellent also in handling property with low viscosity even under a room temperature environment, and a method of producing the polymerisable monomer, a curable composition and a resin member each using the polymerizable monomer.

### Solution to Problem

The above-mentioned object can be achieved by the polymerizable monomer of claim 1, and the method of forming the polymerizable monomer of claim 10.

A polymerizable monomer according to another embodiment of the present invention is preferably represented by the following general formula (2): 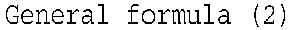 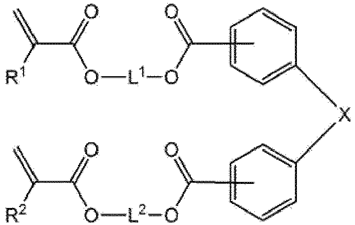 in the general formula (2), X, L¹, L², R^{1,} and R² are the same as those shown in the general formula (1).

A polymerizable monomer according to another embodiment of the present invention is preferably represented by the following general formula (3): 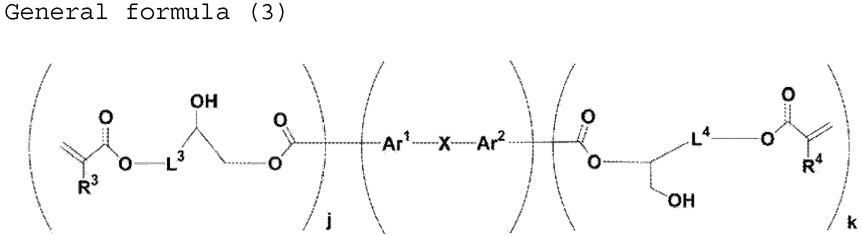 in the general formula (3), X is the same as that shown in the general formula (1), Ar¹ and Ar² are the same as those shown in the general formula (1) except that the valence may be only divalence, L³ and L⁴ each represent a divalent hydrocarbon group having a main chain with a number of atoms within a range of from 1 to 8, and may be identical to or different from each other, R³ and R⁴ each represent a hydrogen atom or a methyl group, j represents 0, 1, or 2, k represents 0, 1, or 2, and j+k=2.

In a polymerizable monomer according to another embodiment of the present invention, a combination (j, k) of values j and k shown in the general formula (3) preferably includes a combination selected from the group consisting of (1, 1) and (0, 2).

A polymerizable monomer according to another embodiment of the present invention preferably includes two or more kinds of structural isomers in each of which a combination (j, k) of values j and k shown in the general formula (3) is selected from the group consisting of (2, 0), (1, 1), and (0, 2).

In a polymerizable monomer according to another embodiment of the present invention, an average value of values k in all molecules of the polymerizable monomer is preferably 0.05 or more and less than 2.0.

The above-mentioned object is also achieved by the method of producing a polymerizable monomer according to claim 10, the curable composition according to claim 11, and the resin member according to claim 12.

### Advantageous Effects of Invention

According to the present invention, the polymerizable monomer excellent in mechanical strength of its cured product and excellent also in handling property with low viscosity even under a room temperature environment, and the method of producing the polymerisable monomer, the curable composition and the resin member each using the polymerizable monomer can be provided.

### Description of Embodiments

A polymerizable monomer according to this embodiment is represented by the following general formula (1): 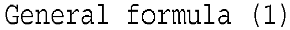 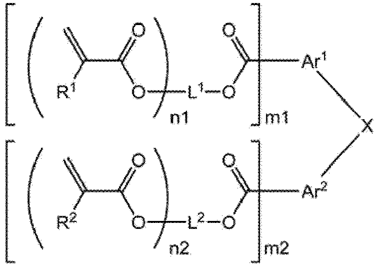 in the general formula (1), X represents a divalent group, Ar¹ and Ar² each represent an unsubstituted aromatic group having a valence selected from divalence to tetravalence, and may be identical to or different from each other, L¹ and L² each represent a substituted or unsubstituted hydrocarbon group having a main chain with a number of atoms within a range of from 2 to 60 and having a valence selected from divalence to tetravalence, and may be identical to or different from each other, R¹ and R² each represent a hydrogen atom or a methyl group, and m1, m2, n1, and n2 each represent an integer selected from a range of from 1 to 3, wherein the divalent group X is -O-. The polymerizable monomer represented by the general formula (1) may be an isomeric mixture containing two or more kinds of isomers.

The polymerizable monomer according to this embodiment is excellent in mechanical strength of its cured product, and is also excellent in handling property because of having low viscosity even under a room temperature environment. The reasons why such effects are obtained are not clear, but the inventors of the present invention presume as follows. First, a possible reason for being excellent in mechanical strength of the cured product is that the polymerizable monomer has structure containing an aromatic group having high rigidity (Ar¹-X-Ar²) at the central portion of the molecule.

In addition, a first possible reason why the polymerizable monomer according to this embodiment shows low viscosity even under a room temperature environment is that the structure (Ar¹-X-Ar²) in which the aromatic group Ar¹ and the aromatic group Ar² are bonded to each other through the divalent group X is adopted as the structure of the central portion of the molecule instead of the structure (Ar¹-Ar²) in which the aromatic group Ar¹ and the aromatic group Ar² are directly bonded to each other through a σ-bond like biphenyl structure or the like. It is considered that although the structure (Ar¹-Ar²) is liable to cause crystallization owing to high symmetry of the molecular structure, the structure (Ar¹-X-Ar²) having the divalent group X further introduced into such structure increases the flexibility of the molecular structure to decrease the symmetry, resulting in a decrease in crystallinity, to thereby decrease the viscosity. Inaddition, in the polymerizable monomer according to this embodiment, the ester bonds linked to the aromatic groups Ar¹ and Ar² are considered to greatly contribute to the decrease in viscosity.

In addition, as described above, the structure (Ar¹-X-Ar²) exhibits higher flexibility of the molecular structure than the structure (Ar¹-Ar²) does. Accordingly, the polymerizable monomer according to this embodiment provides a cured product that is not liable to become brittle and that is also excellent in bending strength, as compared to a polymerizable monomer having the structure (Ar¹-Ar²) introduced into the central portion of the molecule.

Next, the polymerizable monomer represented by the general formula (1) is described in more detail. First, in the general formula (1), Ar¹ and Ar² each represent an aromatic group having a valence selected from divalence to tetravalence, and may be identical to or different from each other.

Specific examples of the aromatic groups Ar¹ and Ar² include divalent to tetravalent benzenes represented by the following structural formulae Ar-a1 to Ar-a3, divalent to tetravalent naphthalenes represented by the following structural formulae Ar-a4 to Ar-a6, and divalent to tetravalent anthracenes represented by the following structural formulae Ar-a7 to Ar-a9. In these structural formulae, bonding sites may each be located on any carbon of the benzene rings constituting the aromatic groups Ar¹ and Ar² (except for carbon forming a condensed portion between benzene rings) . For example, in the case of the structural formula Ar-a1 (divalent benzene), the two bonding sites may be located at ortho positions, meta positions, or para positions.

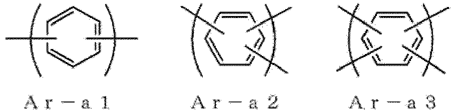

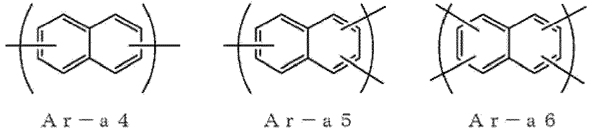

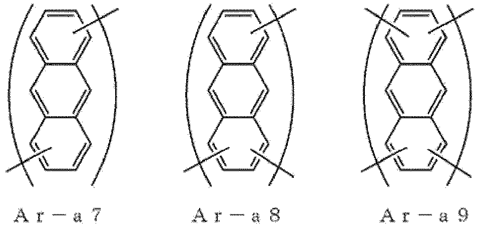

The valence of the aromatic group Ar¹ is determined on the basis of the number represented by m1, and is represented by m1+1. Similarly, the valence of the aromatic group Ar² is determined on the basis of the number represented by m2, and is represented by m2+1.

The aromatic groups Ar¹ and Ar² are unsubstituted.

X represents a divalent group crosslinking the aromatic group Ar¹ and the aromatic group Ar², as exemplified by the following structural formulae X-1.

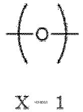

The divalent group X is an electron donating group capable of donating an electron to the benzoate structure (electron withdrawing group) formed of the aromatic group Ar¹ (or Ar²) and the ester bond linked thereto. The divalent group X is -O-. With the electron donating divalent group X, resonance structures as exemplified in the following resonance structural formula are possible, and hence the central portion of the molecule has relatively high polarity. Accordingly, affinity for a hydrophilic material having high polarity can be further improved, and as a result, it becomes easy to improve compatibility with the hydrophilic material and improve affinity and adhesive property for a hydrophilic surface . The following resonance structural formula represents the central portion of the molecule of the polymerizable monomer according to this embodiment in the case where the divalent group X is -O- and the aromatic groups Ar¹ and Ar² are each a phenylene group (provided that its two bonding sites are located at para positions).

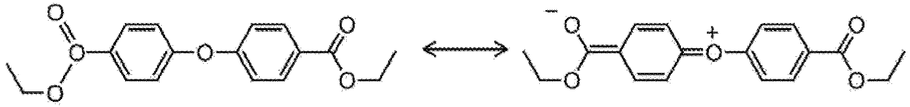

L¹ and L² each represent a hydrocarbon group having a main chain with a number of atoms within the range of from 2 to 60 and having a valence selected from divalence to tetravalence, and may be identical to or different from each other. The number of atoms of the main chain falls within preferably the range of from 2 to 12, more preferably the range of from 2 to 10, still more preferably the range of from 2 to 6, particularly preferably the range of from 2 to 3. Particularly when the number of atoms of the main chain is set to fall within the range of from 2 to 3, the bending strength of the cured product is further increased with ease.

The atoms constituting the main chain are basically constituted of carbon atoms and all the atoms may be carbon atoms, but part of the carbon atoms constituting the main chain may be substituted by a heteroatom. Examples of the heteroatom may include an oxygen atom, a nitrogen atom, a sulfur atom, and a silicon atom. When the main chain contains an oxygen atom as the heteroatom, the main chain may have an ether bond or an ester bond introduced thereinto . The number of heteroatoms that may be introduced into the main chain is preferably half or less of the number of atoms of the main chain. When the number of atoms of the main chain is 2, the number of heteroatoms that may be introduced into the main chain is 1.

In addition, of the atoms constituting the main chain, at least any one atom (generally a carbon atom) may have bonded thereto a substituent. Examples of such substituent may include: an alkyl group having 1 to 3 carbon atoms, such as a methyl group; a hydroxy group; a monovalent hydrocarbon group having a hydroxy group; a halogen atom; -COOR⁴; and -OR⁴. R⁴ is the same as the alkyl group having 1 to 3 carbon atoms. In addition, the number of carbon atoms of the monovalent hydrocarbon group having a hydroxy group falls within preferably the range of from 1 to 3, more preferably the range of from 1 to 2. Specific examples of the monovalent hydrocarbon group having a hydroxy group include -CH₂OH, -CH₂CH₂OH, and -CH(CH₃)OH.

When it is desired to improve compatibility with a hydrophilic material or improve affinity for a hydrophilic surface, it is preferred that at least any one of L¹ and L² contain a hydroxy group, in other words, at least any one of L¹ and L² have, as a substituent, a hydroxy group and/or a monovalent hydrocarbon group having a hydroxy group. The number of hydroxy groups contained in each of L¹ and L² may be at least 1 or more, but is generally preferably 1. In addition, it is more preferred that L¹ and L² each contain one hydroxy group, and in this case, when m1 and m2=1, two hydroxy groups are contained in the molecule.

In general, a compound having a plurality of hydroxy groups in the molecule forms intermolecular hydrogen bonding, and as a result, is liable to have increased viscosity. Therefore, also when the polymerizable monomer according to this embodiment has hydroxy groups in the molecule, the viscosity is liable to increase. However, the case where a hydroxy group is present in the vicinity of the ester bond directly bonded to the aromatic group Ar¹ or Ar² is more preferred because the increase in viscosity is relatively suppressed. Herein, the "case where a hydroxy group is present in the vicinity of the ester bond" specifically means the case where any one or both of L¹ and L² have a hydroxy group and the number of atoms of the main chain of L¹ or L² having a hydroxy group falls within the range of from 2 to 10. The number of atoms of the main chain particularly preferably falls within the range of from 2 to 3. The reason why the above-mentioned effect is obtained is not clear, but the inventors of the present invention assume as follows.

In the case where a hydroxy group is present in the vicinity of the ester bond directly bonded to the aromatic group Ar¹ or Ar², it is predicted that as shown in the structural formula below, the hydrogen of the hydroxy group present in the divalent group L¹ or L² easily forms intramolecular hydrogen bonding between itself and the oxygen of the carbonyl group of the ester bond directly bonded to the aromatic group Ar¹ or Ar². The structural formula exemplified below is an example of the general formula (1) where Ar¹ and Ar²=phenylene group, X=-O-, L¹ and L²=-CH₂CH(OH)CH₂-, R¹ and R²=methyl group, and m1, m2, n1, and n2=1.

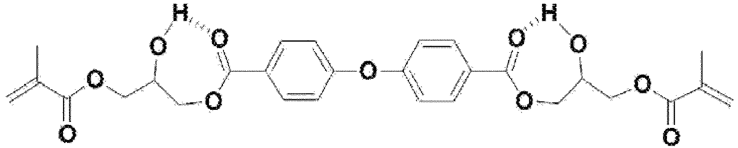

That is, when intramolecular hydrogen bonding is formed, the formation of intermolecular hydrogen bonding is suppressed. Accordingly, as compared to the case where the polymerizable monomer according to this embodiment contains no hydroxy group in the molecule, in the case where a hydroxy group is contained in the molecule, the viscosity increases, but such a significant increase in viscosity as to reach a viscosity comparable to that of a related-art polymerizable monomer having a hydroxy group in the molecule (e.g., Bis-GMA) is suppressed.

In addition, as compared to the case where no intramolecular hydrogen bonding is formed, in the case where intramolecular hydrogen bonding is formed, distortion occurs in the benzoate structure formed of the benzene ring constituting the aromatic group Ar¹ or Ar² and the ester bond directly bonded thereto, and thus the symmetry of the molecular structure at the central portion of the molecule decreases. Therefore, it is considered that the crystallinity of the molecule decreases to further suppress the significant increase in viscosity. The formation of intramolecular hydrogen bonding weakens intermolecular bonding, and hence has a risk of causing a decrease in mechanical strength of the cured product as well. However, when the polymerizable monomer according to this embodiment has a hydroxy group in the vicinity of the ester bond directly bonded to the aromatic group Ar¹ or Ar², the hydroxy group is, more accurately, considered to contribute to intramolecular hydrogen bonding to a relatively higher degree than to intermolecular hydrogen bonding, and is considered to contribute also to forming a loose hydrogen bonding network between molecules. Further, when a plurality of hydroxy groups are contained in the molecule, e.g., when L¹ and L² both have a hydroxy group, the formation of a hydrogen bonding network having high density between molecules is facilitated. In this case, it is considered that as compared to the polymerizable monomer according to this embodiment having no hydroxy group in the molecule, the mechanical strength of the cured product can be further increased.

Specific examples of L¹ and L² when n1 and n2=1 (when L¹ and L² each represent a divalent hydrocarbon group) may include the following structural formulae L-b1 to L-b14. Of the two bonding sites shown in each of these structural formulae, the bonding site marked with symbol "*" means a bonding site to be bonded to the oxygen atom of the ester bond constituting the benzoate structure at the central portion of the molecule. In the following structural formulae L-b1 to L-b14, a represents an integer selected from the range of from 1 to 11, b represents an integer selected from the range of from 1 to 19, c represents an integer selected from the range of from 0 to 11, d represents an integer selected from the range of from 0 to 5, e represents an integer selected from the range of from 2 to 5, and f represents an integer selected from the range of from 1 to 6. The values of a to f are preferably selected so that the number of atoms of the main chain in each of the structural formulae L-b1 to L-b14 is 12 or less.

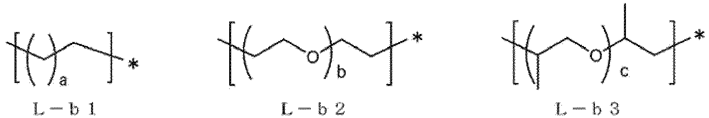

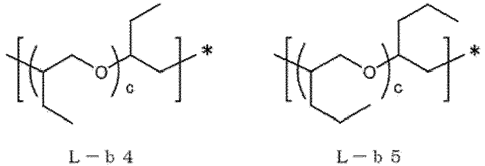

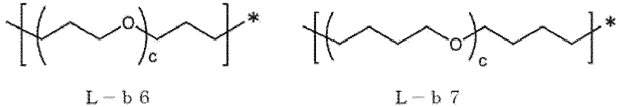

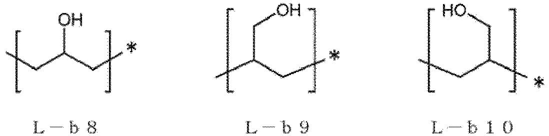

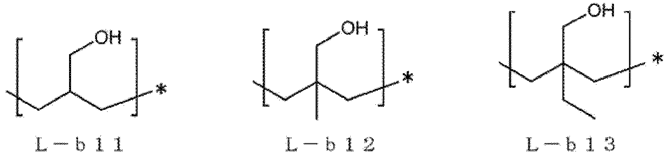

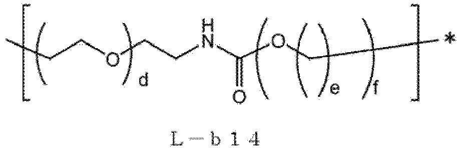

Meanwhile, when n1 and n2=2 (when L¹ and L² each represent a trivalent hydrocarbon group), of the carbon atoms constituting the main chain in each of the structural formulae L-b1 to L-b14, the carbon atom at the farthest position from the carbon atom having the bonding site marked with symbol "*" has two bonding sites. In addition, when n1 and n2=3 (when L¹ and L² each represent a tetravalent hydrocarbon group), of the carbon atoms constituting the main chain in each of the structural formulae L-b1, L-b2, L-b6 to L-b8, and L-b10 to L-b14, the carbon atom at the farthest position from the carbon atom having the bonding site marked with symbol "*" has three bonding sites.

The polymerizable monomer according to this embodiment, which is represented by the general formula (1), is particularly preferably a polymerizable monomer represented by the following general formula (2) . The general formula (2) represents the structure of the general formula (1) in the case where m1, m2, n1, and n2=1, and Ar¹ and Ar²=-C₆H₄- (structural formula Ar-a1).

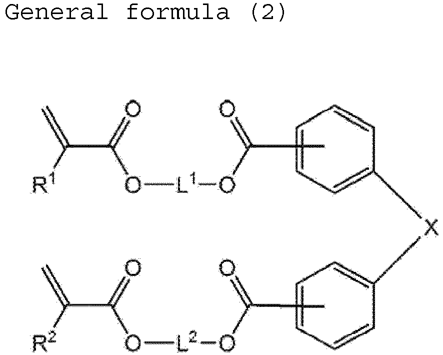

Inaddition, thepolymerizablemonomer according to this embodiment is preferably a polymerizable monomer represented by the following general formula (3). In the general formula (3), X is the same as that shown in the general formula (1), Ar¹ and Ar² are the same as those shown in the general formula (1) except that the valence may be only divalence, L³ and L⁴ each represent a divalent hydrocarbon group having a main chain with a number of atoms within the range of from 1 to 8, and may be identical to or different from each other, and R³ and R⁴ each represent a hydrogen atom or a methyl group. In addition, j represents 0, 1, or 2, k represents 0, 1, or 2, and j+k=2. The general formula (3) represents the structure (bifunctional structure) of the general formula (1) in the case where m1, m2, n1, and n2=1, L¹=-L³-CH(OH)CH₂- or -CH(CH₂OH)-L⁴-, L²=-L³-CH(OH)CH₂- or -CH(CH₂₀H)-L⁴-, R¹ corresponds to R³ or R⁴, and R² corresponds to R³ or R⁴. In addition, in the general formula (3), the groups shown in parentheses on both left and right sides may each be bonded to any of the two bonding sites of the group shown in the center: -Ar¹-X-Ar²-. That is, depending on the values of j and k, the group shown in the parentheses on the left side in the general formula (3) is bonded to both sides of the group shown in the center in some cases, and the group shown in the parentheses on the right side in the general formula (3) is bonded to both sides of the group shown in the center in other cases.

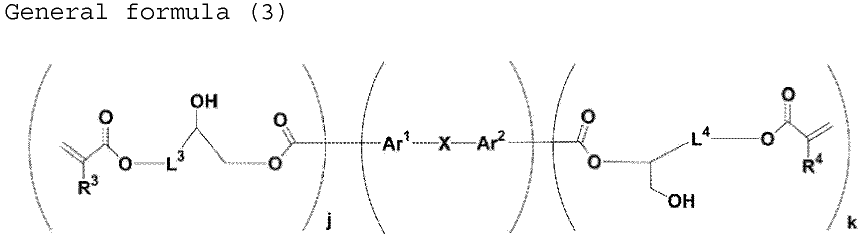

In each of L³ and L⁴, the atoms constituting the main chain are basically constituted of carbon atoms and all the atoms may be carbon atoms, but part of the carbon atoms constituting the main chain may be substituted by a heteroatom. Examples of the heteroatom may include an oxygen atom, a nitrogen atom, a sulfur atom, and a silicon atom. When the main chain contains an oxygen atom as the heteroatom, the main chain may have an ether bond or an ester bond introduced thereinto. The number of heteroatoms that may be introduced into the main chain is preferably 1 or 2. When the number of atoms of the main chain is 2, the number of heteroatoms that may be introduced into the main chain is 1.

In addition, in each of L³ and L⁴, the number of atoms of the main chain only needs to be from 1 to 8, but is more preferably from 1 to 5, still more preferably from 1 to 3, most preferably 1. Specific examples of L³ and L⁴ include: an alkylene group having a main chain with 1 to 8 carbon atoms, such as a methylene group, an ethylene group, a n-propylene group, or a n-butylene group; and a group obtained by partially or completely substituting the main chain of the alkylene group with an ether bond or an ester bond (provided that the number of atoms of the main chain of the alkylene group is 2 or more).

As the combination (j, k) of the values j and k shown in the general formula (3), there are given (2, 0), (1, 1), and (0, 2). Of those, from the viewpoint that the suppression of the decomposition of the polymerizable monomer molecule can be expected, (1, 1) and (0, 2) are more preferred. With regard to the reason for this, the inventors of the present invention assume as follows.

Here, the formula shown below represents an example of a mechanism for a reaction of the molecule of the polymerizable monomer represented by the general formula (3) where (j, k)=(1, 1), X=-O-, Ar¹ and Ar²=-C₆H₄-, L³ and L⁴=-CH₂-, and R³ and R⁴=-CH₃ in the presence of a water molecule. The polymerizable monomer according to this embodiment has molecular structure having an ester bond directly bonded to an aromatic group at the central portion of the molecule. One possible reaction mechanism by which the polymerizable monomer having such molecular structure is decomposed is, for example, a nucleophilic reaction of a water molecule on an ester bond directly bonded to an aromatic group (hydrolysis reaction) (Route B below) . However, when a primary alcohol contained in the same molecule is present in the immediate vicinity of the ester bond directly bonded to the aromatic group, the primary alcohol and the ester bond cause a cyclization reaction in the molecule, to thereby temporarily form cyclic structure (Route A below) . The cyclization reaction is reversible, and hence the cyclic structure easily returns to the original linear structure immediately, but the cyclization reaction inhibits the above-mentioned nucleophilic reaction (hydrolysis reaction) or the like. Accordingly, it is assumed that the decomposition of the polymerizable monomer molecule is inhibited. In this case, decomposition deterioration of the polymerizable monomer or a composition containing the polymerizable monomer is suppressed (in other words, storage stability is improved), and hence the initial performance of any of various compositions using the polymerizable monomer immediately after production can be stably maintained over a long period of time. For example, in the case of a dental composition (e.g. , a dental filling restorative material or a dental adhesive) using the polymerizable monomer, its adhesive strength immediately after production and the mechanical strength of its cured product are easily maintained even after long-term storage.

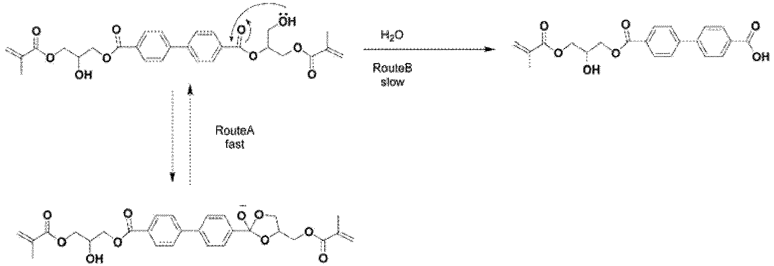

In addition, the polymerizable monomer according to this embodiment preferably contains two or more kinds of structural isomers in each of which the combination (j, k) of the values j and k shown in the general formula (3) is selected from the group consisting of (2, 0), (1, 1), and (0, 2). When the polymerizable monomer contains two or more kinds of structural isomers for the combination of (j, k) shown in the general formula (3), it is easy to improve the mechanical strength of the cured product and the storage stability in a well-balanced manner. In this case, the average value of the values k in all molecules of the polymerizable monomer preferably falls within the range of from 0.05 or more to less than 2.0 (in other words, the average value of the values j falls within the range of from more than 0 to 1.95 or less) . Further, the lower limit of the average value of the values k is preferably 0.1 or more, and the upper limit of the average value of the values k is more preferably 1.7 or less, still more preferably 1.5 or less, particularly preferably 0.4 or less. In order to improve the mechanical strength of the cured product and the storage stability in a well-balanced manner, the value k=2 (state of not containing structural isomers) is suitable as well as the case where the average value of the values k is set to the range of from 0.05 or more to less than 2.0. In the state of containing two or more kinds of structural isomers, the mechanical strength in the initial state without undergoing a certain storage period can be made higher than in the case of the value k=2 (state of not containing structural isomers) . In this regard, it is more advantageous that the average value of the values k is smaller within the range in which the average value of the values k is not less than 0.05.

When only the above-mentioned reaction mechanism is taken into consideration, it is predicted that as the average value of the values k increases, the storage stability also improves. However, an investigation made by the inventors of the present invention has found that a remarkable improving effect on the storage stability is obtained even when the average value of the values k is about 0.1 (when the presence ratio of the primary alcohol present in the vicinity of an ester bond directly bonded to an aromatic group is small in the whole polymerizable monomer) (see Examples to be described later) . From those results, it is assumed that an unexpected factor other than the above-mentioned reaction mechanism exists for the improvement of the storage stability.

The polymerizable monomer according to this embodiment may be synthesized by appropriately combining known starting raw materials and a known synthesis reaction method, and its production method is not particularly limited. For example, when the polymerizable monomer represented by the general formula (3) is produced, there may be utilized a production method including at least a reaction step of allowing a compound represented by the following general formula (4) and a compound represented by the following general formula (5) to react with each other. In this case, a polymerizable monomer containing two or more kinds of structural isomers selected from the group consisting of compounds represented by the following general formulae (6) to (8) may be produced.

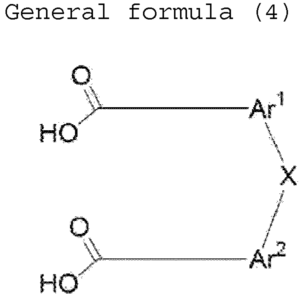

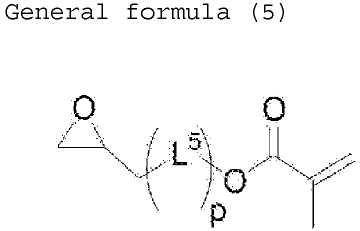

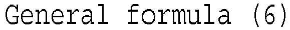

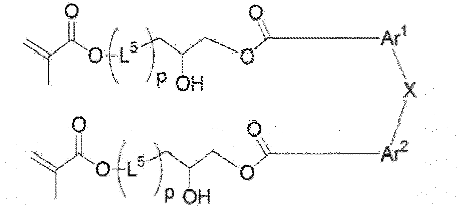

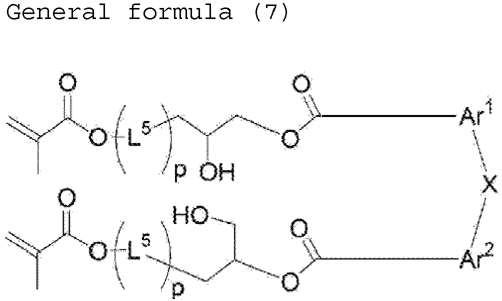

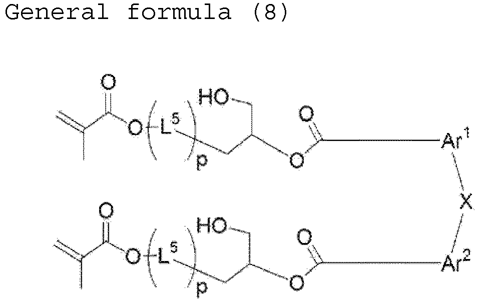

In the general formulae (4) to (8), X, Ar¹, and Ar² are the same as those shown in the general formula (3), and L⁵ represents a divalent hydrocarbon group having a main chain with a number of atoms of from 1 to 7. In addition, p represents 0 or 1. Here, the average value of the values k, in other words, the presence ratio among the structural isomers represented by the general formulae (6) to (8) canbe easily adjusted by appropriately selecting synthesis conditions. In addition, as necessary, through purification treatment after the synthesis, the average value of the values k (presence ratio among the structural isomers represented by the general formulae (6) to (8)) may be adjusted so as to be closer to a desired value.

In L⁵ shown in the general formulae (5) to (8), the atoms constituting the main chain are basically constituted of carbon atoms and all the atoms may be carbon atoms, but part of the carbon atoms constituting the main chain may be substituted by a heteroatom. Examples of the heteroatom may include an oxygen atom, a nitrogen atom, a sulfur atom, and a silicon atom. When the main chain contains an oxygen atom as the heteroatom, the main chain may have an ether bond or an ester bond introduced thereinto . The number of heteroatoms that may be introduced into the main chain is preferably 1 or 2. When the number of atoms of the main chain is 2, the number of heteroatoms that may be introduced into the main chain is 1.

In addition, in L⁵, the number of atoms of the main chain only needs to be from 1 to 7, but is preferably from 1 to 4, more preferably 1 or 2. Specific examples of L⁵ include: an alkylene group having a main chain with 1 to 7 carbon atoms, such as a methylene group, an ethylene group, a n-propylene group, and a n-butylene group; and a group obtained by partially or completely substituting the main chain of the alkylene group with an ether bond or an ester bond (provided that the number of atoms of the main chain of the alkylene group is 2 or more).

In the general formula (6), L⁵'s may be identical to or different from each other. The same applies to the general formulae (7) and (8). When L⁵'s are set to be different from each other, two or more kinds of compounds different from each other in L⁵ may be used as the compound represented by the general formula (5) to be used for the synthesis. In addition, p preferably represents 0.

If necessary, after the polymerizable monomer containing two or more kinds of structural isomers has been obtained by the above-mentioned production method, only a polymerizable monomer substantially not containing structural isomers (e.g., the polymerizable monomer represented by the general formula (6)) may be isolated and purified. However, the polymerizable monomer to be obtained by the isolation and purification tends to be poor in terms of compatibility between the mechanical strength of the cured product and the storage stability as compared to the polymerizable monomer containing two or more kinds of structural isomers before the isolation and purification treatment. In addition, the isolation and purification treatment is further needed in the production of the polymerizable monomer, and hence is liable to be disadvantageous in terms of cost. Accordingly, from those viewpoints, the isolation and purification treatment is preferably avoided.

### -Composition, Curable Composition, and Resin Member-

The polymerizable monomer according to this embodiment may be utilized as a composition containing the polymerizable monomer. However, from the viewpoint of utilization in various applications, the polymerizable monomer according to this embodiment is also suitably used as a composition containing the polymerizable monomer according to this embodiment and any other material. In this case, a polymerization initiator may be further added to a composition containing at least the polymerizable monomer according to this embodiment, to thereby formulate a curable composition. When the composition containing the polymerizable monomer according to this embodiment is a composition containing no polymerization initiator, this composition (agent A) may be used in combination with another composition (agent B) containing a polymerization initiator. In this case, a cured product may be obtained by polymerizing a mixture of the agent A and the agent B (first curing mode).

In addition, in the case of the curable composition (agent C) containing the polymerizable monomer according to this embodiment and a polymerization initiator, a cured product may be obtained by curing the agent C alone (second curing mode) . However, as necessary, the agent C may be used in combination with another composition (agent D). In this case, a cured product may be obtained by curing a mixture of the agent C and the agent D (third curing mode) . In addition, a cured product may also be obtained by applying the agent D onto a solid surface, and further placing the agent C thereon, followed by curing. In this case, when the agent D functions as an adhesive and/or a pretreatment material for improving conformability between the agent C and the solid surface, a cured product in a state of being bonded to the solid surface can be obtained (fourth curing mode). In addition, when the agent D functions as a release agent, after curing, the cured product can be collected by being easily separated from the solid surface without any damage to the surface of the cured product (fifth curing mode).

As a composition that may be suitably utilized in each of the first and third curing modes, for example, there is given a two-component system adhesive. As a composition that may be suitably utilized in the second curing mode, for example, there is given a resin raw material to be used for a 3D printer utilizing a layered manufacturing method. As a composition that maybe suitably utilized in the fourth curing mode, for example, there is given a dental material (in particular, a composite resin to be used for filling the cavity of a tooth) . As a composition that may be suitably utilized in the fifth curing mode, for example, there is given a resin raw material to be used for the production of a molded product using a mold.

In addition, the other material to be used together with the polymerizable monomer according to this embodiment is not particularly limited, and may be appropriately selected depending on applications of the composition or curable composition containing the polymerizable monomer according to this embodiment. Specific examples of the other material may include a polymerizable monomer other than the polymerizable monomer according to this embodiment, a low-molecular organic compound having no reactivity, a resin, a filler, an organic-inorganic composite material, various additives other than the above-mentioned polymerization initiator, and a solvent. Two or more kinds of those materials may be used in combination.

However, the other material to be used in combination with the polymerizable monomer according to this embodiment is particularly preferably another polymerizable monomer. As the other polymerizable monomer, a known polymerizable monomer may be used without any limitation. However, in consideration of the fact that the polymerizable monomer according to this embodiment has a feature of having low viscosity under a room temperature environment, it is preferred that the other polymerizable monomer also have such relatively low viscosity as not to offset the feature. From such viewpoint, the viscosity of the other polymerizable monomer is preferably 150 mPa·S or less at room temperature (25°C). Further, in consideration of the ease of ensuring compatibility when reactive groups located at both ends of a molecule are identical or similar to each other, the other polymerizable monomer is preferably a bifunctional (meth)acrylate-based polymerizable monomer.

Examples of the bifunctional (meth)acrylate-based polymerizable monomer having the above-mentioned viscosity characteristic may include polyalkylene glycol dimethacrylates, such as triethylene glycol dimethacrylate (3G) (more specifically, polyethylene glycol dimethacrylate having a polymerization degree of alkylene glycol units of 1 or more and 14 or less, polypropylene glycol dimethacrylate having a polymerization degree of alkylene glycol units of 1 or more and 7 or less, polymethylene glycol dimethacrylate having 2 to 10 carbon atoms, and the like), neopentyl glycol dimethacrylate, tricyclodecane dimethanol dimethacrylate (TCD), and 1,9-nonanediol dimethacrylate (ND). In addition, from the viewpoint of compatibility, the other polymerizable monomer is preferably one having a polyalkylene glycol chain, more preferably one having a polyethylene glycol chain. This is because the polyalkylene glycol chain has high affinity for the benzoate structure constituting the central portion of the molecule of the polymerizable monomer according to this embodiment (that is, a non-hydrogen-bonding polar group). In consideration of the respects described above, the other polymerizable monomer is preferably polyalkylene glycol dimethacrylate, particularly preferably triethylene glycol dimethacrylate.

In the description of the present application, the viscosity of the polymerizable monomer means a value measured at 25°C using an E-type viscometer.

In addition, the other polymerizable monomer may be selected depending on applications of the composition or curable composition using the polymerizable monomer according to this embodiment, or a resin member including the cured product obtained by using the polymerizable monomer according to this embodiment. For example, when the composition or curable composition using the polymerizable monomer according to this embodiment is used as a dental material, a polymerizable monomer, such as triethylene glycol dimethacrylate, tricyclodecanedimethanol dimethacrylate, or 1,9-nonanediol dimethacrylate, is preferably used. When the polymerizable monomer according to this embodiment and the other polymerizable monomer are used as a mixture, the polymerizable monomer according to this embodiment accounts for preferably 20 mass% or more, more preferably 30 mass% or more, particularly preferably 60 mass% or more of all polymerizable monomers.

As the polymerization initiator to be used in the curable composition using the polymerizable monomer according to this embodiment, a known polymerization initiator may be used, and any of various polymerization initiators, such as a radical-type, cation-type, or anion-type photopolymerization initiator, and an azo-based or peroxide-based thermal polymerization initiator, may be appropriately utilized. For example, when polymerization and curing are performed by light irradiation, a photopolymerization initiator, such as camphorquinone or ethyl p-N,N-dimethylaminobenzoate, may be used. In addition, two or more kinds of those polymerization initiators may be used in combination. Further, any other additive, such as a polymerization inhibitor or a sensitizer, may be used in combination with the polymerization initiator.

In addition, it is also suitable to add a filler to the composition or curable composition using the polymerizable monomer according to this embodiment. The use of the filler can further increase the suppressive effect on polymerization shrinkage. In addition, through the use of the filler, the operability of the composition or curable composition before curing can be improved, or mechanical properties after curing can be improved. As the filler, for example, an inorganic filler formed of particles of an inorganic oxide, such as amorphous silica, silica-titania, silica-zirconia, silica-titania-barium oxide, quartz, or alumina, may be used, and an organic filler or an organic-inorganic composite filler may also be used. In addition, the particle diameter and shape of the filler are not particularly limited, but for example, particles each having a spherical shape or an irregular shape and having an average particle diameter of from about 0.01 µm to about 100 µm may be appropriately used depending on purposes. In addition, any such filler may be treated with a surface treatment agent typified by a silane coupling agent from the viewpoint of improving conformability between the polymerizable monomer according to this embodiment and the other material, such as the other polymerizable monomer, to be used in combination as necessary, to thereby improve mechanical strength and water resistance.

The polymerizable monomer according to this embodiment may be used in various applications, such as a dental material like a dental curable composition or a dental adhesive, an optical material, a printing plate, a photoresist material, a paint, an adhesive, an ink, a stereolithographic resin, a woodworking coating, a hard coating, a film coating, a paper coating, an optical fiber coating, a PVC floor coating, a ceramic wall coating, a resin hard coat, a metallized base coat, a release coating, a metal coating, a glass coating, an inorganic material coating, an elastic coating, a planographic ink, a metal can ink, a screen printing ink, a gravure varnish, calendering, a paint, a sealant, an adhesive for paper, an adhesive for a film, an adhesive for woodworking, an adhesive for an inorganic material, an adhesive for a plastic, a solvent-based adhesive, a water-based adhesive, a hot-melt adhesive, a reaction-type adhesive, a pressure-sensitive adhesive, a sealing agent, a gloss coating agent, an OP varnish, an etching resist, a solder-resist, a dry film, an interlayer adhesion agent for a build-up circuit board, a photosensitive material, a semiconductor photoresist, a printed wiring board resist, a photosensitive flexographic plate, a photosensitive resin relief printing plate, a PS plate, a CTP plate, PDP, a color resist for a color filter, a black matrix material, a color filter overcoat agent, a photoresist for a liquid crystal, a seal agent, an injection port-sealing agent, a prism sheet, potting, a moisture-proof coat, a protective coat, an ink binder, an adhesive binder, a sealing material, a primer for a base material, e.g., a plastic, a metal, or paper, an agent for temporary fixing, a stereolithographic material, a cast resin, a reactive diluent, temporary anticorrosion of iron, a metal primer/overcoat, a primer for a metal coil/sheet, a pressure-sensitive adhesive tape, a transfer film, a plastic lens, and a glass lens. In addition, depending on its use applications, the polymerizable monomer according to this embodiment may also be used by being mixed with various components as necessary. In addition, the cured product obtained by using the composition containing the polymerizable monomer according to this embodiment is excellent in mechanical strength. Accordingly, the resin member including the cured product is also suitably utilized in applications requiring mechanical strength.

Meanwhile, the polymerizable monomer according to this embodiment in which L¹ and L² each have, as a substituent, a hydroxy group and/or a monovalent hydrocarbon group having a hydroxy group, and Bis-GMA are similar to each other in the following three respects : a) both have hydroxy groups; b) the structures of reactive groups at their molecular ends are identical (methacrylic group and methacrylic group) or substantially identical (acrylic group and methacrylic group) ; and c) the central portion of the molecule has molecular structure mainly formed of an aromatic group. However, when attention is focused on the molecular structure at the central portion of the molecule, the polymerizable monomer according to this embodiment has benzoate structure, which has higher polarity than that of the bisphenol A structure constituting the central skeleton of Bis-GMA. Accordingly, it is considered that the polymerizable monomer according to this embodiment in which L¹ and L² each have, as a substituent, a hydroxy group and/or a monovalent hydrocarbon group having a hydroxy group (hereinafter sometimes referred to as "polymerizable monomer according to this embodiment having hydroxy groups") has higher hydrophilicity than that of Bis-GMA. In addition, the polymerizable monomer disclosed in Patent Literature 4 has nohydroxygroup. That is, the polymerizable monomer according to this embodiment having hydroxy groups is hydrophilic, whereas the polymerizable monomer disclosed in Patent Literature 4 is hydrophobic. In addition, in the polymerizable monomer disclosed in Patent Literature 4 or 5, the central portion of the molecule has biphenyl structure having high symmetry and low polarity, but in the polymerizable monomer according to this embodiment having hydroxy groups, the central portion of the molecule has structure having the divalent group X introduced between the two aromatic groups Ar¹ and Ar², which is likely to have higher polarity than that of the biphenyl structure. Therefore, when attention is focused on only the structure of the central portion of the molecule, particularly in the case where the divalent group X is an electron donating group, it is considered that the polymerizable monomer according to this embodiment having hydroxy groups is more hydrophilic than the polymerizable monomer disclosed in Patent Literature 4 or 5.

In consideration of those respects, when compared to Bis-GMA or the polymerizable monomer disclosed in Patent Literature 4 or Patent Literature 5, the polymerizable monomer according to this embodiment having hydroxy groups may be said to be suitable and advantageous for utilization in applications requiring more hydrophilicity. For example, when the polymerizable monomer according to this embodiment having hydroxy groups is used as an adhesive, it is preferred that a surface onto which the adhesive is to be applied be hydrophilic, and when the polymerizable monomer according to this embodiment having hydroxy groups is used by being mixed with any other material, it is preferred that the other material be a hydrophilic material. When an adhesive containing the polymerizable monomer according to this embodiment is used for a hydrophilic surface, more excellent adhesive strength can be obtained as compared to the case of using an adhesive containing Bis-GMA or the polymerizable monomer disclosed in Patent Literature 4 or Patent Literature 5. In addition, when a mixed composition obtained by mixing the polymerizable monomer and a hydrophilic solid material, such as an inorganic filler, which relatively shows hydrophilicity even after subjected to surface treatment with a silane coupling agent or the like, is produced, the polymerizable monomer according to this embodiment facilitates the mixing, and moreover, allows a larger amount of a hydrophilic solid material to be blended and easily suppresses an increase in viscosity of the mixed composition along with the increase in blending amount, as compared to Bis-GMA or the polymerizable monomer disclosed in Patent Literature 4.

A non-aromatic (meth)acrylate-based polymerizable monomer having a hydroxy group, such as pentaerythritol dimethacrylate, is also useful because the polymerizable monomer is excellent in handling property with low viscosity, and is also excellent in adhesive property for a hydrophilic member surface, as compared to Bis-GMA or the polymerizable monomer disclosed in Patent Literature 4 or Patent Literature 5. However, the non-aromatic (meth)acrylate-based polymerizable monomer having a hydroxy group does not have an aromatic skeleton in the molecule, and hence is poor in mechanical strength of its cured product. In addition, such polymerizable monomer has a hydroxy group in the molecule, and hence has high hydrophilicity, but is poor in water resistance, and hence is not suitable for long-term use under water or a high-humidity environment, e.g., in an oral cavity. However, the polymerizable monomer according to this embodiment having hydroxy groups can exhibit excellent characteristics also in terms of the mechanical strength of the cured product and the water resistance as compared to the non-aromatic (meth)acrylate-based polymerizable monomer having a hydroxy group.

In consideration of the characteristics of the polymerizable monomer according to this embodiment described above, the polymerizable monomer can be widely used as a component of a dental curable composition. The polymerizable monomer according to this embodiment having hydroxy groups is particularly suitably used as a polymerizable monomer constituting a dental material, in particular, a dental adhesive to be used for bonding onto a hydrophilic tooth surface, or a dental filling restorative material (composite resin) in which an inorganic filler is blended to further increase mechanical strength and which is required to have high affinity for the tooth surface serving as a hydrophilic adherend surface. Further, the polymerizable monomer according to this embodiment may be used also as a dental adhesive composite resin having the functions of both the dental adhesive and the composite resin.

### Examples

Now, in order to specifically describe the present invention, Examples and Comparative Examples are given and described, but the present invention is by no means limited thereto. Abbreviations or symbols of substances used in the preparation of samples of Examples and Comparative Examples, and their structural formulae or substance names, preparation methods for various samples, and various evaluation methods are described below.

### (1) Abbreviations or Symbols, and their Structural Formulae or Substance Names

### [First Polymerizable Monomer (Polymerizable Monomer represented by General Formula (1))]

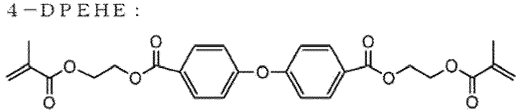

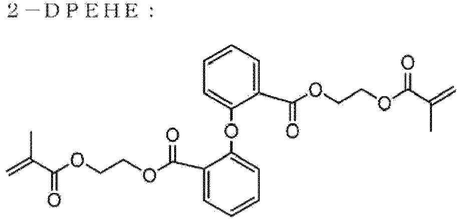

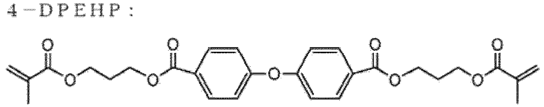

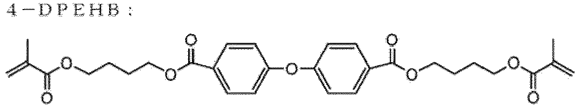

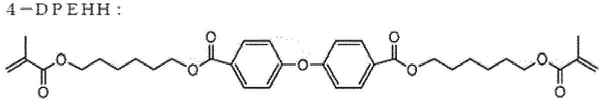

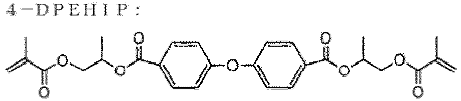

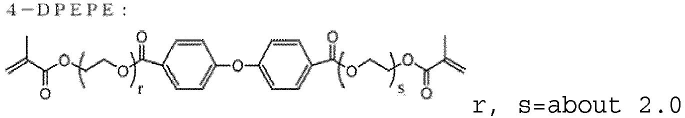

In the structural formulae, the numbers r and s of repetitions of unit structures may each take an integer value of 0 or more in individual molecules, and the structural formulae are each a mixture of two or more kinds of polymerizable monomer molecules having different combinations of integer values (r, s). 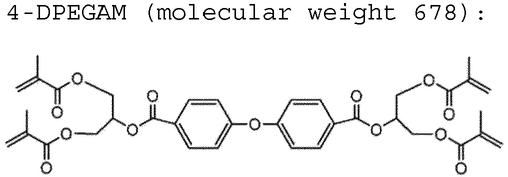 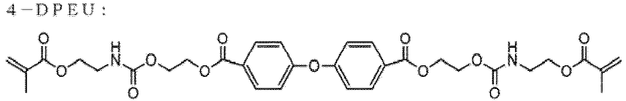 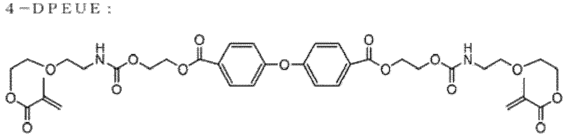 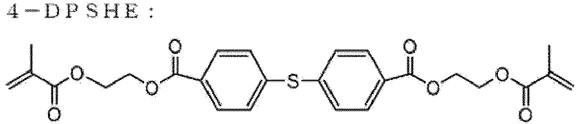 (For reference only, not according to the invention) 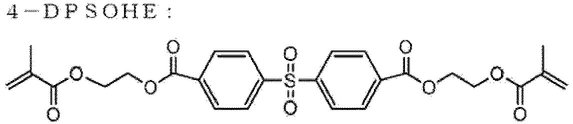 (For reference only, not according to the invention) 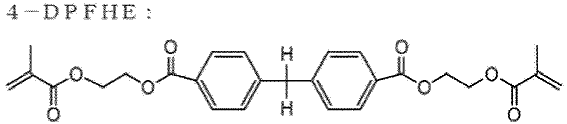 (For reference only, not according to the invention) 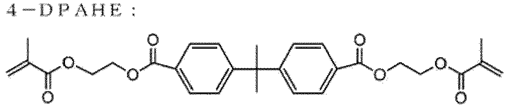 (For reference only, not according to the invention) 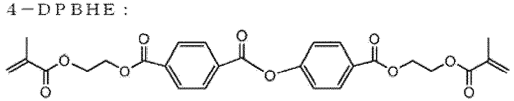 (For reference only, not according to the invention) 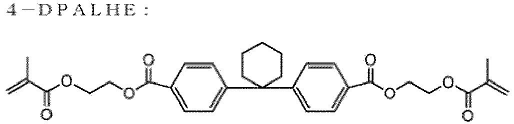 (For reference only, not according to the invention) 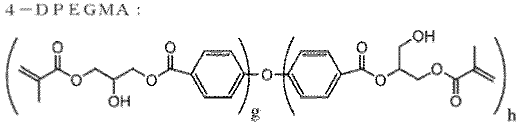 g=1.6 (average value), h=0.4 (average value)

4-DPEGMA is obtained as a mixture of the following compounds (a), (b), and (c), and their ratio is 65:30:5 in terms of molar ratio. In addition, the values g and h shown with the structural formula above are average values of the mixture of the compounds (a), (b), and (c). The values g and h shown with the structural formula above and the following structural formulae mean average values, but in individual molecules, the values of g and h may each take an integer value of 0, 1, or 2. In addition, except for the case where the average values of the values g and h are each 0 or 2, the structural formulae in which the average values of the values g and h are shown each mean a mixture of two kinds or three kinds of structural isomers having different combinations of integer values (g, h). Further, in the general formula (3), in the case of Ar¹=Ar²=phenylene group, the value g is a value corresponding to the value j shown in the general formula (3), and the value h is a value corresponding to the value k shown in the general formula (3). 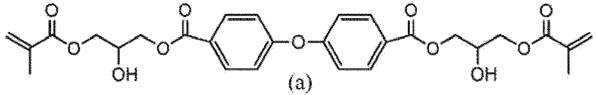 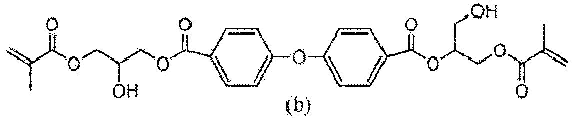 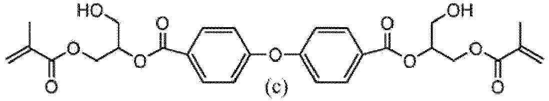 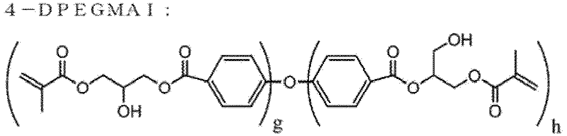 g=1.9 (average value), h=0.1 (average value) 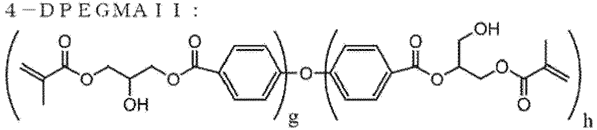 g=1.8 (average value), h=0.2 (average value) 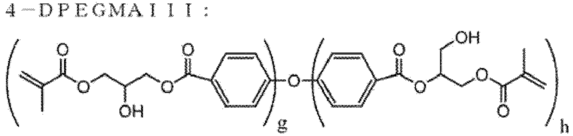 g=2.0 (average value), h=0 (average value) 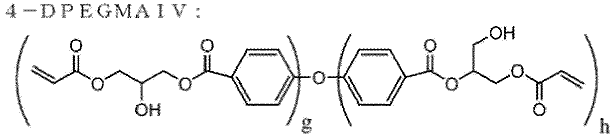 g=0.4 (average value), h=1.6 (average value) 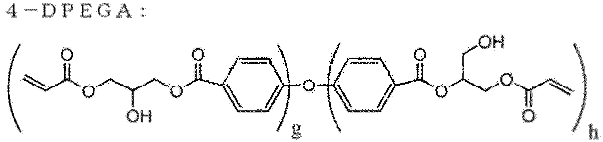 g=1.6 (average value), h=0.4 (average value) 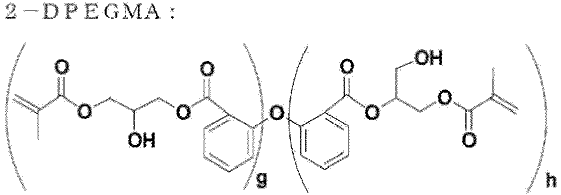 g=1.6 (average value), h=0.4 (average value) 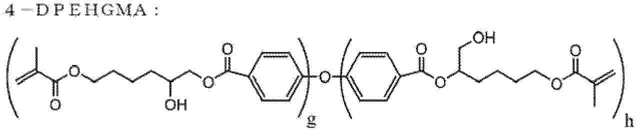 g=1.6 (average value), h=0.4 (average value) 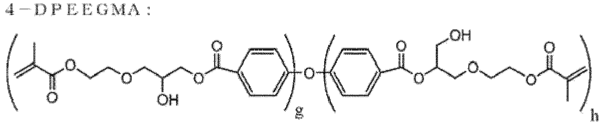 g=1.6 (average value), h=0.4 (average value) 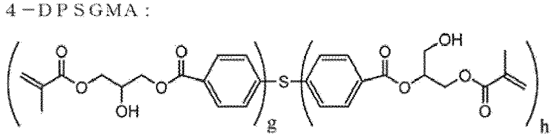 g=1.6 (average value), h=0.4 (average value) (For reference only, not according to the invention) 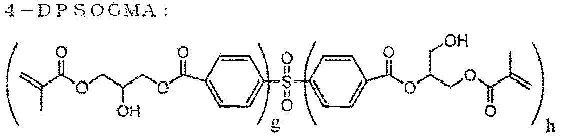 g=1.6 (average value), h=0.4 (average value) (For reference only, not according to the invention) 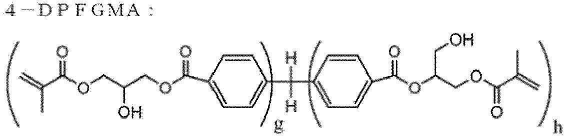 g=1.6 (average value), h=0.4 (average value) (For reference only, not according to the invention) 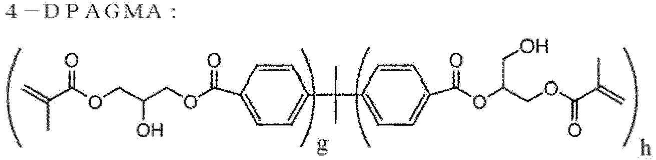 g=1.6 (average value), h=0.4 (average value) (For reference only, not according to the invention) 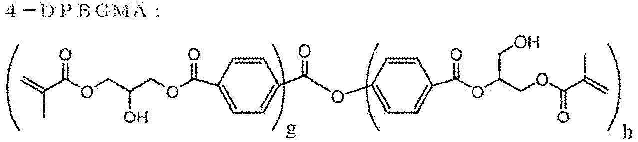 g=1.6 (average value), h=0.4 (average value) (For reference only, not according to the invention) 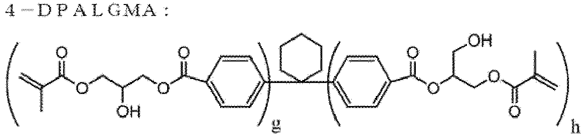 g=1.6 (average value), h=0.4 (average value) (For reference only, not according to the invention)

### [First Polymerizable Monomer (Polymerizable Monomer having Molecular Structure other than General Formula (1))]

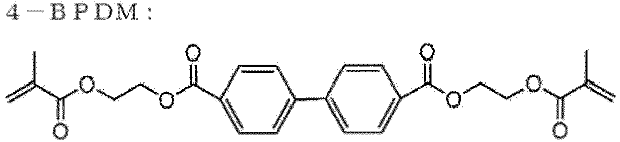

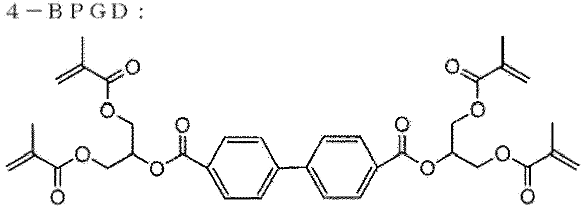

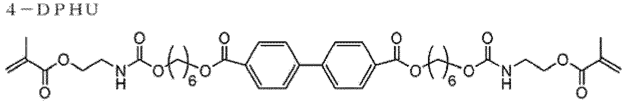

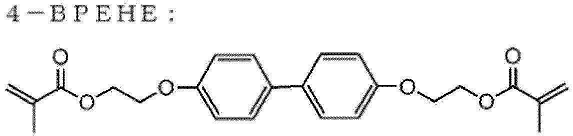

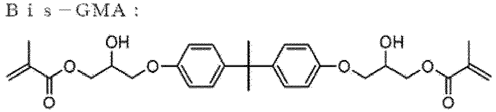

### [Second Polymerizable Monomer]

3G: triethylene glycol dimethacrylate
ND: 1,9-nonanediol dimethacrylate
TCD: tricyclodecane dimethanol dimethacrylate
PM: mixture obtained by mixing 2-methacryloyloxyethyl dihydrogen phosphate and bis(2-methacryloyloxyethyl) hydrogen phosphate at a molar ratio of 1:1
HEMA: 2-hydroxyethyl methacrylate

### [Photopolymerization Initiator]

CQ: camphorquinone
DMBE: ethyl p-N,N-dimethylaminobenzoate

### [Polymerization Inhibitor]

BHT: 2,6-di-t-butyl-p-cresol

### [Inorganic Filler]

F1: mixture obtained by mixing spherical silica-zirconia (average particle diameter: 0.4 µm) subjected to hydrophobizing treatment with γ-methacryloyloxypropyltrimethoxysilane, and spherical silica-zirconia (average particle diameter: 0.07 µm) subjected to hydrophobizing treatment with γ-methacryloyloxypropyltrimethoxysilane at a mass ratio of 70:30.

### (2) Viscosity Measurement

The viscosity of a polymerizable monomer was measured using a CS rheometer. A measuring apparatus used was a viscoelasticity measuring apparatus CS rheometer "CVO120HR" (manufactured by Bohlin Instruments Ltd.) including a 4 cm/2° cone/plate geometry and a temperature control system. In addition, measurement was performed three times under the measurement conditions of a measurement temperature (plate temperature) of 25°C and a shear rate of 1 rps, and the average value of the measured values of the three times was defined as a viscosity.

### (3) Preparation of Matrix Monomer Sample

A first polymerizable monomer and a second polymerizable monomer were mixed at a predetermined mass ratio shown in Table 1 to Table 3. With respect to 100 parts by mass of the mixture of those polymerizable monomers, 0.5 part by mass of CQ, 0.8 part by mass of DMBE, and 0.1 part by mass of BHT were added, and then the mixture was stirred in the dark until becoming homogeneous. Thus, a matrix monomer sample was obtained.

### (4A) Preparation of Curable Composition Sample for Bending Strength Evaluation

As shown in Table 1 to Table 3, 101.4 parts by mass of the above-mentioned matrix monomer sample and 205.9 parts by mass of the inorganic filler F1 were weighed out, and mixed in an agate mortar to provide a mixture. Subsequently, the mixture was degassed under vacuum to remove air bubbles, to thereby provide a paste-like curable composition sample for bending strength evaluation. The inorganic filler-containing curable composition is one that may be utilized as a dental curable composition, preferably a composite resin. As shown in Table 1 to Table 3, some matrix monomer samples were also degassed under vacuum to remove air bubbles without being mixed with the inorganic filler, to thereby prepare curable composition samples for bending strength evaluation.

In the preparation of the curable composition sample, in the case of using Bis-GMA as the first polymerizable monomer, at the time of weighing, Bis-GMA was weighed in a state of being heated to 60°C in an incubator. In addition, in the case of using 4-BPDM, 4-BPGD, 4-DPHU, and 4-BPHE as the first polymerizable monomers, when any such first polymerizable monomer and the second polymerizable monomer were mixed, the mixing was performed while heating was performed with a dryer. Meanwhile, when the curable composition sample was prepared using the polymerizable monomer represented by the general formula (1) as the first polymerizable monomer, the entire process of the preparation work was performed under a room temperature environment (25°C).

### (4B) Measurement of Bending Strength of Cured Product

In accordance with a bending strength measurement method for a dental filling composite resin of Class 2, Group 1 specified by JIS T6514:2013, each sample was subjected to visible light irradiation with a visible light irradiator (TOKUSO POWER LITE manufactured by Tokuyama Corporation) for 20 seconds to prepare a cured product sample, and its bending strength was measured. A universal testing machine (Autograph manufactured by Shimadzu Corporation) was used for the measurement.

The measurement of bending strength was performed for each of: a cured product sample (initial) obtained by curing the curable composition sample immediately after preparation; and a cured product sample (after storage at 60°C) obtained by curing the prepared curable composition sample after storage under an environment of 60°C in an incubator for 5 months. The measurement of the bending strength after storage at 60°C was performed for only some kinds of cured product samples.

### (5A) Preparation of Curable Composition Sample for Adhesive Strength Test

A first polymerizable monomer and a second polymerizable monomer were mixed at a predetermined mass ratio shown in Table 4. Next, with respect to 100 parts by mass of the mixture of those polymerizable monomers, 1.5 parts by mass of CQ, 1.5 parts by mass of DMBE, 10 parts by mass of purified water, 85 parts by mass of acetone, and 0.3 part by mass of BHT were added, and then the mixture was stirred in the dark until becoming homogeneous . Thus, a curable composition sample for an adhesive strength test was obtained. The curable composition sample is one that may be utilized as a dental adhesive (so-called bonding material).

### (5B) Adhesive Strength Test Methods

As an adhesive strength test, evaluation was performed using a member having a surface showing hydrophilicity as a bonding object. In this test, as the member having a surface showing hydrophilicity, a tooth having dentin exposed to its surface was selected. The dentin is a hydrophilic material containing hydroxyapatite, water, and other organic substances. Details of adhesive strength test methods are described below.

### (5B-1) Initial Adhesive Strength

A bovine front tooth extracted within 24 hours after killing was polished with waterproof abrasive paper P600 under a flow of water to carve out a dentin plane so as to be parallel to a labial surface and flat. Next, the carved-out plane was dried by being blown with compressed air for about 10 seconds. Next, a double-sided tape having a hole having a diameter of 3 mm was bonded onto the plane, and further, a paraffin wax having a thickness of 0.5 mm and having a hole having a diameter of 8 mm was fixed with the center of the hole of the paraffin wax arranged on the center of the hole of the previously bonded double-sided tape, to thereby form a simulated cavity. To the simulated cavity, the curable composition sample for an adhesive strength test (curable composition sample immediately after preparation) was applied, left to stand for 20 seconds, and then dried by being blown with compressed air for about 10 seconds. Visible light irradiation was performed for 10 seconds with a visible light irradiator (TOKUSO POWER LITE manufactured by Tokuyama Corporation) to cure the curable composition sample. Further, a composite resin (ESTELITE SIGMA QUICK manufactured by Tokuyama Dental Corporation) was filled thereon, and the resultant was pressed with a polyester sheet. After the filling, the resultant was similarly cured by being irradiated with visible light for 10 seconds to prepare a bonded test piece.

Onto the composite resin cured body top surface of the bonded test piece, resin cement (BISTITE II manufactured by Tokuyama Dental Corporation) was applied, and an attachment made of SUS having a cylindrical shape having a diameter of 8 mm and a length of 25 mm was further bonded. Next, the resin cement was cured at 37°C for 15 minutes, and then the bonded test piece was immersed in water at 37°C for 24 hours, to thereby provide a bonded test piece I with an attachment. After that, the bonded test piece I with an attachment was pulled using a universal testing machine (Autograph manufactured by Shimadzu Corporation) at a crosshead speed of 1 mm/min, and tensile adhesive strength between the tooth and the composite resin cured body was measured. The tensile adhesive strength was measured for each of eight test pieces prepared for each Example or Comparative Example. Then, the average value of the tensile adhesive strengths of the eight times was defined as the initial adhesive strength of each Example or Comparative Example.

### (5B-2) Adhesive Strength after Endurance Test

The bonded test piece I with an attachment was put into a thermal shock tester, and subjected to an endurance test involving repeating the following operations 3,000 times: the test piece was immersed in a water bath at 4°C for 1 minute, then transferred to a water bath at 60°C and immersed therein for 1 minute, and returned to the water bath at 4°C again. After that, for the bonded test piece I subjected to the endurance test, tensile adhesive strength was measured in the same manner as in the case of determining the initial adhesive strength, and was defined as adhesive strength after an endurance test.

### (5B-3) Adhesive Strength after Storage at 60°C

Adhesive strength was measured by the procedure described in (5B-1) except that the prepared curable composition sample that had been stored in an incubator under an environment of 60°C for 20 days was used inplace of the curable composition sample immediately after preparation used in the measurement of the initial adhesive strength.

### (6) Synthesis Procedure for Polymerizable Monomer

Of the first polymerizable monomers used in the preparation of the matrix monomer samples, ones corresponding to the polymerizable monomer represented by the general formula (1) were synthesized by the following procedures.

### <Synthesis of Acid Chloride Product (A) >

A first mixed liquid formed of 25.3 g (0.096 mol) of 4,4'-diphenyletherdicarboxylic acid, 0.85 g (0.012 mol) of dimethylformamide, and 80 ml of toluene was prepared. To the first mixed liquid in a stirred state, a second mixed liquid formed of 58.4 g (0.46 mol) of thionyl chloride and 20 ml of toluene was slowly added dropwise under room temperature. After the completion of the dropwise addition, the resultant liquid was heated to 95°C and refluxed for 3 h. Then, after the heating and reflux, the resultant yellow transparent liquid was allowed to cool to provide a toluene solution of 4,4'-diphenyletherdicarboxylic acid chloride having the molecular structure shown below (hereinafter sometimes referred to as "acid chloride product (A)"). Further, the toluene solution was subjected to a rotary evaporator to remove toluene, thionyl chloride, and hydrogen chloride at 40°C, and thus 26.9 g of 4,4'-diphenyletherdicarboxylic acid chloride (0.091 mol, percentage yield: 95%) was obtained as a solid.

### Acid chloride product (A)

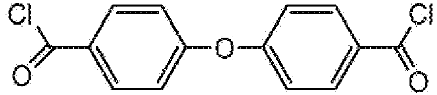

### <Synthesis of 4-DPEHE>

To 15.3 g (0.052 mol) of the acid chloride product (A), 120 ml of methylene chloride was added to provide a dispersion liquid containing the acid chloride product (A). A mixed liquid obtained by mixing 16.9 g (0.13 mol) of 2-hydroxyethyl methacrylate, 7.7 g (0.13 mol) of triethylamine, 0.16 g (0.0013 mol) of 4-dimethylaminopyridine, 0.002 g of BHT, and 10 ml of methylene chloride was slowly added dropwise to the dispersion liquid of the acid chloride product (A) at -78°C through the utilization of a dropping funnel, and the mixture was further stirred for 5 hours. After the dropwise addition and the stirring, water was added to the resultant liquid, and the solvent was removed using a rotary evaporator. After the solvent removal, the resultant residue was dissolved in 100 ml of toluene, and the solution was washed with a 0.5 N hydrochloric acid solution, washed with brine, and dried over magnesium sulfate, followed by separation by filtration. The resultant filtrate was concentrated with a rotary evaporator, and then the concentrated liquid was further vacuum-dried to provide 4-DPEHE (yield: 19.0 g, percentage yield: 76%, HPLC purity: 97%) . ¹H NMR spectrum data on the resultant 4-DPEHE was as follows. ¹H NMR δ 1.93 (s, 6H), 4.58 (t, 4H), 4.63 (t, 4H), 5.59 (s, 2H), 6.14 (s, 2H), 7.06 (d, 4H), 7.96 (d, 4H)

### <Synthesis of 2-DPEHE>

27.8 g of 2,2'-diphenyletherdicarboxylic acid chloride (0.094 mol, percentage yield: 98%) was obtained by the same method as in the case of the synthesis of the acid chloride product (A) except that 25.3 g (0.096 mol) of 2,2'-diphenyletherdicarboxylic acid was used in place of 25.3 g of 4,4'-diphenyletherdicarboxylic acid.

2-DPEHE (yield: 19.5 g, percentage yield: 78%, HPLC purity: 97%) was obtained by the same method as in the case of the synthesis of 4-DPEHE except that 15.3 g (0.052 mol) of 2,2'-diphenyletherdicarboxylic acid chloride was used in place of 15.3 g (0.052 mol) of the acid chloride product (A). ¹H NMR spectrum data on the resultant 2-DPEHE was as follows.
¹H NMR δ 1.93 (s, 6H), 4.54 (t, 4H), 4.62 (t, 4H), 5.58 (s, 2H), 6.16 (s, 2H), 7.06 (d, 4H), 7.45 (d, 2H), 7.96 (d, 2H)

### <Synthesis of 4-DPEHP>

8.6 g (0.1 mol) of methacrylic acid, 15.2 g (0.2 mol) of 1,3-propanediol, 0.86 g (0.005 mol) of p-toluenesulfonic acid, and 0.1 g of BHT serving as a polymerization inhibitor were loaded into a glass container, heated to 85°C, and stirred. The pressure in the reaction system in the heated and stirred state was reduced, and while water was removed from the reaction system, stirring was continued for 5 hours. After that, the resultant liquid was cooled, purified using silica gel column chromatography, and concentrated. Thus, 6.3 g of 3-hydroxypropyl methacrylate (percentage yield: 44%) was obtained.

Next, 3.0g (0.01 mol) of the acid chloride product (A), 70 ml of methylene chloride, and 0.001 g of di-tert-butylmethylphenol were loaded into another glass container, and while the resultant solution was stirred, to the solution, a solution obtained by dissolving 3.2 g (0.022 mol) of the above-mentioned 3-hydroxypropyl methacrylate, 2.0 g (0.02 mol) of triethylamine, and 0.025 g (0.0002 mol) of 4-dimethylaminopyridine in 10 ml of methylene chloride was slowly added dropwise over 1 hour. After the completion of the dropwise addition, the resultant solution was stirred at room temperature for 1 hour, and then water was added, followed by the removal of the solvent using a rotary evaporator. After the solvent removal, the resultant residue was dissolved in 100 ml of toluene, and the solution was washed with a 0.5 N hydrochloric acid solution, washed with brine, and dried over magnesium sulfate, followed by separation by filtration. The resultant filtrate was concentrated again with a rotary evaporator, and then the concentrated liquid was further vacuum-dried to provide 4-DPEHP (yield: 4.1g, percentage yield: 76%, HPLC purity: 97%). ¹H NMR spectrum data on the resultant 4-DPEHP was as follows.
¹H NMR δ 1.93 (s, 6H), 2.10 (m, 4H), 4.18 (t, 4H), 4.27 (t, 4H), 5.59 (s, 2H), 6.13 (s, 2H), 7.05 (d, 4H), 7.93 (d, 4H)

### <Synthesis of 4-DPEHB>

### -Process 1-

8.6 g (0.1 mol) of methacrylic acid, 18.0 g (0.2 mol) of 1,4-butanediol, 0.86 g (0.005 mol) of p-toluenesulfonic acid, and 0.1 g of BHT serving as a polymerization inhibitor were loaded into a glass container, heated to 85°C, and stirred. Next, the pressure in the reaction system in the heated and stirred state was reduced, and while water was removed from the reaction system, stirring was continued for 5 hours. After that, the resultant liquid was cooled, purified using silica gel column chromatography, and concentrated. Thus, 6.7 g of 4-hydroxybutyl methacrylate (percentage yield: 42%) was obtained.

### -Process 2-

Next, 3.0 g (0.01 mol) of the acid chloride product (A), 70 ml of methylene chloride, and 0.001 g of di-tert-butylmethylphenol were loaded into another glass container, and while the resultant solution was stirred, to the solution, a solution obtained by dissolving 3.5 g (0.022 mol) of the above-mentioned 4-hydroxybutyl methacrylate, 2.0 g (0.02 mol) of triethylamine, and 0.025 g (0.0002 mol) of 4-dimethylaminopyridine in 10 ml of methylene chloride was slowly added dropwise over 1 hour. After the completion of the dropwise addition, the resultant solution was stirred at room temperature for 1 hour, and then water was added, followed by the removal of the solvent using a rotary evaporator. After the solvent removal, the resultant residue was dissolved in 100 ml of toluene, and the solution was washed with a 0.5 N hydrochloric acid solution, washed with brine, and dried over magnesium sulfate, followed by separation by filtration. The resultant filtrate was concentrated again with a rotary evaporator, and then the concentrated liquid was further vacuum-dried to provide 4-DPEHB (yield: 4.1g, percentage yield: 76%, HPLC purity: 97%). ¹H NMR spectrum data on the resultant 4-DPEHB was as follows.
¹H NMR δ 1.52-1.63 (m, 8H), 1.93 (s, 6H), 4.14 (t, 4H), 4.27 (t, 4H), 5.59 (s, 2H), 6.13 (s, 2H), 7.05 (d, 4H), 7.93 (d, 4H)

### <Synthesis of 4-DPEHH>

19.7 g (percentage yield: 53%) or 16.0 g (percentage yield: 43%) of 6-hydroxyhexyl methacrylate was obtained in accordance with the synthesis method shown in Process 1 of <Synthesis of 4-DPEHB> except that 23.6 g of 1,6-hexanediol was used in place of 18.0 g of 1,4-butanediol.

Then, 4-DPEHH (yield: 4.6g, percentage yield: 78%, HPLC purity: 98%) was obtained through a reaction between 6-hydroxyhexyl methacrylate and the acid chloride product (A) in accordance with the synthesis method shown in Process 2 of <Synthesis of 4-DPEHB> except that 4.1 g of 6-hydroxyhexyl methacrylate was used in place of 4-hydroxybutyl methacrylate. ¹H NMR spectrum data on the resultant 4-DPEHH was as follows.
¹H NMR δ 1.29 (m, 8H), 1.57 (t, 4H), 1.77 (t, 4H), 1.93 (s, 6H), 4.16 (t, 4H), 4.22 (t, 4H), 5.58 (s, 2H), 6.14 (s, 2H), 7.04 (d, 4H), 7.96 (d, 4H)

### <4-DPEHIP>

4-DPEHIP (yield: 19.9 g, percentage yield: 75%, HPLC purity: 97%) was synthesized by the same method as the synthesis method for 4-DPEHE except that 18.7 g (0.13 mol) of 2-hydroxypropyl methacrylate was used in place of 16.9 g of 2-hydroxyethyl methacrylate. ¹H NMR spectrum data on the resultant 4-DPEHIP was as follows.
¹H NMR δ 1.40 (d, 6H), 1.93 (s, 6H), 4.61 (d, 4H), 4.74 (t, 2H), 5.56 (s, 2H), 6.13 (s, 2H), 7.01 (d, 4H), 7.92 (d, 4H)

### <4-DPEPE>

4-DPEPE (yield: 22.5 g, percentage yield: 78%, HPLC purity: 97%) was synthesized by the same method as the synthesis method for 4-DPEHE except that 22.6 g (0.13 mol) of polyethylene glycol monomethacrylate (average of numbers n of repetitions of ethylene glycol chains (-CH₂CH₂O-)n≈2) was used in place of 16.9 g of 2-hydroxyethyl methacrylate. ¹H NMR spectrum data on the resultant 4-DPEPE was as follows.
¹H NMR δ 1.93 (s, 6H), 3.64-3.82 (m, 8H), 4.36-4.42 (m, 8H), 5.58 (s, 2H), 6.17 (s, 2H), 7.04 (d, 4H), 7.99 (d, 4H)

### <Synthesis of 4-DPEGAM>

4-DPEGAM (yield: 25.7 g, percentage yield: 73%, HPLC purity: 92%) was synthesized by the same method as the synthesis method for 4-DPEHE except that 29.6 g (0.13 mol) of glycerol dimethacrylate was used in place of 16.9 g of 2-hydroxyethyl methacrylate. ¹H NMR spectrum data on the resultant 4-DPEGAM was as follows.
¹H NMR δ 1.93 (s, 12H), 4.58 (d, 8H), 5.20 (m, 2H), 5.52 (s, 4H), 6.10 (s, 4H), 7.01 (d, 4H), 7.92 (d, 4H)

### <Synthesis of 4-DPEU>

50.0 g (0.8 mol) of ethylene glycol, 40.5 g (0.4 mol) of triethylamine, and 0.49 g (4 mol) of N,N-dimethylaminopyridine were dissolved in 100 ml of methylene chloride, and the resultant solution was cooled to 0°C while being stirred. Next, to the solution, a solution obtained by dissolving 44.8g (0.2mol) of the acid chloride product (A) in methylene chloride (200ml) was slowly added dropwise over 2 hours. After the completion of the dropwise addition, the resultant solution was further stirred for 1 hour, and then water was added, followed by the removal of the solvent using a rotary evaporator. After the solvent removal, the resultant residue was dissolved in 100 ml of toluene, and the solution was washed with a 0.5 N hydrochloric acid solution, washed with brine, and dried over magnesium sulfate, followed by separation by filtration. The resultant filtrate was again concentrated with a rotary evaporator, and then the concentrated liquid was purified by silica gel column chromatography to provide 41.6 g of 4,4'-bis(2-hydroxyethoxycarbonyl)diphenyl ether (percentage yield: 60%).

34.6 g (0.1 mol) of the resultant 4,4'-bis(2-hydroxyethoxycarbonyl)diphenyl ether and 3.2g (5 mmol) of dibutyltin dilaurate were dissolved in 100 ml of anhydrous dimethylformamide. To the resultant solution, 31.0 g (0.2 mol) of 2-methacryloyloxyethyl isocyanate was further added, and the mixture was stirred at room temperature for 3 hours. To the solution after the stirring, 100 ml of methylene chloride was added. The mixture was washed three times with distilled water through the use of a separatory funnel, and the methylene chloride layer was dried using magnesium sulfate. After the drying, the magnesium sulfate was filtered off, and the filtrate was concentrated with a rotary evaporator. The concentrate was further vacuum-dried to provide 4-DPEU (yield: 63.7 g, percentage yield: 97%, HPLC purity: 94%). ¹H NMR spectrum data on the resultant 4-DPEU was as follows. ¹H NMR δ 1.93 (s, 6H), 3.30 (t, 4H), 4.40-4.56 (m, 12H), 5.60 (s, 2H), 6.17 (s, 2H), 7.06 (d, 4H), 7.98 (d, 4H), 8.03 (s, 2H)

### <Synthesis of 4-DPEUE>

4-DPEUE (yield: 70.3 g, percentage yield: 95%, HPLC purity: 96%) was obtained by the same method as the synthesis method for 4-DPEU except that 39.8 g (0.2 mol) of 2-(2-methacryloyloxyethyloxy)ethyl isocyanate was used in place of 31.0 g of 2-methacryloyloxyethyl isocyanate. ¹H NMR spectrum data on the resultant 4-DPEUE was as follows.
¹H NMR δ 1.93 (s, 6H), 3.10 (m, 4H), 3.66 (m, 8H), 4.33 (t, 4H), 4.54 (m, 8H), 5.58 (s, 2H), 6.14 (s, 2H), 7.06 (d, 4H), 7.96 (d, 4H), 8.02 (s, 2H)

### <Synthesis of 4-DPSHE (for reference only, not according to the invention)>

48.4 g (0.2 mol) of 4,4-diformyldiphenyl sulfide synthesized by a synthesis method disclosed in JP 2005-154379 A was dissolved in 200 ml of t-butanol and 50 ml of water. After that, 50 ml of an aqueous solution of sodium hydrogen phosphate and 140 g (2 mol) of 2-methyl-2-butene were added, and 36 g (0.4 mol) of sodium chlorite was further added to prepare a reaction solution. Next, the reaction solution was stirred for 5 hours, and then the reaction solution was rendered acidic using a 1 N hydrochloric acid solution to precipitate a solid. Subsequently, the reaction solution having the solid precipitated therein was subjected to suction filtration, and then the precipitated solid was washed with water. After the washing, the resultant solid (compound) was vacuum-dried to provide 4,4'-dicarboxydiphenyl sulfide (yield: 45.5 g, percentage yield: 83%) .

Next, 28.4 g (0.091 mol) of 4,4'-diphenyl sulfide dicarboxylic acid chloride was synthesized by the same method as the synthesis method for the acid chloride product (A) except that 26.4 g (0.096 mol) of 4,4'-dicarboxydiphenyl sulfide was used in place of 25.3 g (0.096 mol) of 4,4'-diphenyletherdicarboxylic acid.

Then, 4-DPSHE (yield: 18.9 g, percentage yield: 73%, HPLC purity: 91%) was obtained by the same method as the synthesis method for 4-DPEHE except that 16.1 g (0.052 mol) of 4,4'-diphenyl sulfide dicarboxylic acid chloride was used in place of 15.3 g of the acid chloride product (A). ¹H NMR spectrum data on the resultant 4-DPSHE was as follows.
¹H NMR δ 1.93 (s, 6H), 4.52 (t, 4H), 4.63 (t, 4H), 5.58 (s, 2H), 6.11 (s, 2H), 7.30 (d, 4H), 7.81 (d, 4H)

### <Synthesis of 4-DPSOHE (for reference only, not according to the invention)>

31.6 g (0.092 mol) of 4,4'-diphenyl sulfone dicarboxylic acid chloride was synthesized by the same method as the synthesis method for the acid chloride product (A) except that 29.4 g (0.096 mol) of 4,4'-dicarboxydiphenyl sulfone was used in place of 25.3 g (0.096 mol) of 4,4'-diphenyletherdicarboxylic acid.

Then, 4-DPSOHE (yield: 20.4 g, percentage yield: 74%, HPLC purity: 92%) was obtained by the same method as the synthesis method for 4-DPEHE except that 17.8 g (0.052 mol) of 4,4'-diphenyl sulfone dicarboxylic acid chloride was used in place of 15.3 g of the acid chloride product (A). ¹H NMR spectrum data on the resultant 4-DPSOHE was as follows.
¹H NMR δ 1.93 (s, 6H), 4.58 (t, 4H), 4.63 (t, 4H), 5.59 (s, 2H), 6.14 (s, 2H), 8.06 (d, 4H), 8.20 (d, 4H)

### <Synthesis of 4-DPFHE (for reference only, not according to the invention)>

44.8 g (0.2 mol) of 4,4'-diformyldiphenylmethane synthesized by a synthesis method disclosed in JP 2005-154379 A was dissolved in 200 ml of t-butanol and 50 ml of water. After that, 50 ml of an aqueous solution of sodium hydrogen phosphate and 140 g (2 mol) of 2-methyl-2-butene were added, and 36 g (0.4 mol) of sodium chlorite was further added to provide a reaction solution. Next, the reaction solution was stirred for 5 hours, and then the reaction solution was rendered acidic using a 1 N hydrochloric acid solution to precipitate a solid. Subsequently, the reaction solution having the solid precipitated therein was subjected to suction filtration, and then the precipitated solid was washed with water. The resultant solid (compound) was vacuum-dried to provide 39.8 g of 4,4'-dicarboxydiphenylmethane (percentage yield: 83%).

Next, 25.2 g (0.091 mol) of 4,4'-diphenylmethane dicarboxylic acid chloride was synthesized by the same method as the synthesis method for the acid chloride product (A) except that 23.0 g (0.096 mol) of 4,4'-dicarboxydiphenylmethane was used in place of 25.3 g (0.096 mol) of 4,4'-diphenyletherdicarboxylic acid.

Then, 4-DPFHE (yield: 18.7 g, percentage yield: 75%, HPLC purity: 94%) was obtained by the same method as the synthesis method for 4-DPEHE except that 14.4 g (0.052 mol) of 4,4'-diphenylmethane dicarboxylic acid chloride was used in place of 15.3 g of the acid chloride product (A). ¹H NMR spectrum data on the resultant 4-DPFHE was as follows.
¹H NMR δ 1.93 (s, 6H), 3.85 (s, 2H), 4.52-4.63 (m, 8H), 5.58 (s, 2H), 6.12 (s, 2H), 7.15 (d, 4H), 7.86 (d, 4H)

### <Synthesis of 4-DPAHE (for reference only, not according to the invention)>

29.6g of 2,2-bis(4-chlorocarbonylphenyl)propane(percentage yield: 96%) was obtained by the same method as the synthesis method for the acid chloride product (A) except that 27.2 g (0.096 mol) of 2,2'-bis(4-carboxyphenyl)propane synthesized by a synthesis method disclosed in GB 753384 A was used in place of 25.3 g of 4,4'-diphenyletherdicarboxylic acid.

Then, 4-DPAHE (yield: 19.3 g, percentage yield: 73%, HPLC purity: 92%) was obtained by the same method as the synthesis method for 4-DPEHE except that 16.7 g (0.052 mol) of 2,2'-bis(4-chlorocarbonylphenyl)propane was used in place of 15.3 g of the acid chloride product (A). ¹H NMR spectrum data on the resultant 4-DPAHE was as follows.
¹H NMR δ 1.64 (s, 6H), 1.93 (s, 6H), 4.53-4.64 (m, 8H), 5.59 (s, 2H), 6.12 (s, 2H), 7.22 (d, 4H), 7.90 (d, 4H)

### <Synthesis of 4-DPBHE (for reference only, not according to the invention)>

50.8 g (0.2 mol) of 4-formylphenyl-4'-formylbenzoate synthesized by a synthesis method disclosed in JP 2007-106779 A was dissolved in 200 ml of t-butanol and 50 ml of water. After that, 50 ml of an aqueous solution of sodium hydrogen phosphate and 140 g (2 mol) of 2-methyl-2-butene were added, and 36 g (0.4 mol) of sodium chlorite was further added to provide a reaction solution. Next, the reaction solution was stirred for 5 hours, and then the reaction solution was rendered acidic using a 1 N hydrochloric acid solution to precipitate a solid. Subsequently, the reaction solution having the solid precipitated therein was subjected to suction filtration, and then the precipitated solid was washed with water. The resultant solid (compound) was vacuum-dried to provide 45.4 g of 4-carboxyphenyl-4'-carboxybenzoate (percentage yield: 84%) .

Next, 29.8 g of 4-chlorocarbonylphenyl-4'-chlorocarbonylphenylbenzoate (percentage yield: 96%) was obtained by the same method as the synthesis method for the acid chloride product (A) except that 25.9 g (0.096 mol) of the above-mentioned 4-carboxyphenyl-4'-carboxybenzoate was used in place of 25.3 g of 4,4'-diphenyletherdicarboxylic acid.

Then, 4-DPBHE (yield: 20.2 g, percentage yield: 76%, HPLC purity: 94%) was obtained by the same method as the synthesis method for 4-DPEHE except that 16.8 g (0.052 mol) of 4-chlorocarbonylphenyl-4'-chlorocarbonylphenylbenzoate was used in place of 15.3 g of the acid chloride product (A). ¹H NMR spectrum data on the resultant 4-DPBHE was as follows.
¹H NMR δ 1.93 (d, 6H), 4.51 (m, 4H), 4.61 (t, 4H), 5.58 (d, 2H), 6.13 (d, 2H), 7.25 (d, 2H), 8.02 (d, 2H), 8.12 (d, 2H), 8.272 (d, 2H)

### <Synthesis of 4-DPALHE (for reference only, not according to the invention)>

58.4 g (0.2 mol) of 1,1-bis(4-formylphenyl)cyclohexane synthesized by a synthesis method disclosed in JP 3076603 B2 was dissolved in 200 ml of t-butanol and 50 ml of water. After that, 50 ml of an aqueous solution of sodium hydrogen phosphate and 140 g (2 mol) of 2-methyl-2-butene were added, and 36 g (0.4 mol) of sodium chlorite was further added to provide a reaction solution. Next, the reaction solution was stirred for 5 hours, and then the reaction solution was rendered acidic using a 1 N hydrochloric acid solution to precipitate a solid. Subsequently, the reaction solution having the solid precipitated therein was subjected to suction filtration, and then the precipitated solid was washed with water. The resultant solid (compound) was vacuum-dried to provide 54.4 g of 1,1-bis(4-carboxylphenyl)cyclohexane (percentage yield: 84%) .

Next, 33.3 g of 1,1-bis(4-chlorocarbonylphenyl)cyclohexane (percentage yield: 96%) was obtained by the same method as the synthesis method for the acid chloride product (A0) except that 31.1 g (0.096 mol) of the above-mentioned 1,1'-bis(4-carboxylphenyl)cyclohexane was used in place of 25.3 g of 4,4'-diphenyletherdicarboxylic acid.

Then, 4-DPALHE (yield: 24.3 g, percentage yield: 85%, HPLC purity: 92%) was obtained by the same method as the synthesis method for 4-DPEHE except that 18.8 g (0.052 mol) of 1,1-bis(4-chlorocarbonylphenyl)cyclohexane was used in place of 15.3 g of the acid chloride product (A). ¹H NMR spectrum data on the resultant 4-DPALHE was as follows.
¹H NMR δ 1.45 (m, 6H), 1.93 (s, 6H), 2.01 (m, 4H), 4.53-4.64 (m, 4H), 5.58 (s, 2H), 6.13 (s, 2H), 7.23 (d, 4H), 7.90 (d, 4H)

### <Synthesis of 4-DPEGMA>

A mixed liquid obtained by adding, to 12.8 g (0.09 mol) of glycidyl methacrylate, 12.9 g (0.05 mol) of 4,4'-diphenyletherdicarboxylic acid, 0.02 g (0.00009 mol) of benzyltriethylammonium chloride, 0.02 g (0.00009 mol) of BHT, and 20 g of dimethylformamide was subjected to a reaction at 100°C for 4 hours. To the liquid obtained by the reaction, 40 ml of ethyl acetate was added, and the mixture was turned into a homogeneous solution. Next, the solution was transferred to a separatory funnel and washed three times with 40 ml of a 10 wt% aqueous solution of potassium carbonate. The resultant was further washed three times with distilled water, and then an ethyl acetate layer was collected. After that, magnesium sulfate was added to the collected ethyl acetate layer to remove the water contained in the ethyl acetate layer. Subsequently, the magnesium sulfate was filtered off from the ethyl acetate layer, and the filtrate was concentrated with a rotary evaporator to provide a concentrate. The concentrate was further vacuum-dried to provide 4-DPEGMA (yield: 22.8 g, percentage yield: 84%, HPLCpurity: 95%). ¹H NMR spectrum data on the resultant 4-DPEGMA was as follows.
¹H NMR δ 1.93 (s, 6H), 3.90 (d, 0.8H), 4.30-4.70 (m, 9.2H), 5.59 (s, 2H), 6.16 (s, 2H), 7.07 (d, 4H), 8.07 (d, 4H)

### <Synthesis of 4-DPEGMAI>

A methylene chloride solution was prepared by dissolving 15.3 g (0.052 mol) of the acid chloride product (A) in a solid state in 30 ml of methylene chloride.

Separately, a solution was prepared by mixing 33.3 g (0.208 mol) of glycerol-1-yl monomethacrylate, 12.1 g (0.104 mol) of tetramethylethylenediamine, and 0.002 g of BHT with 20 ml of methylene chloride. The solution was slowly added dropwise to the methylene chloride solution having dissolved therein the acid chloride A described above at -78°C, and the mixture was further stirred for 5 hours. After the dropwise addition and the stirring, the resultant liquid was washed three times with 60 ml of 0.4 mol/L aqueous hydrochloric acid, then washed three times with 60 ml of a 10 wt% aqueous solution of potassium carbonate, and further washed three times with distilled water, followed by collecting of a methylene chloride layer. After that, magnesium sulfate was added to the collected methylene chloride layer to remove water contained in the methylene chloride layer. Next, the magnesium sulfate was filtered off from the methylene chloride layer, and the filtrate was concentrated with a rotary evaporator to provide a concentrate. The concentrate was purified by silica gel column chromatography to provide 4-DPEGMAI (yield: 25.9 g, percentage yield: 92%, HPLC purity: 97%). ¹H NMR spectrum data on the resultant 4-DPEGMAI was as follows.
¹H NMR δ 1.93 (s, 6H), 3.90 (d, 0.2H), 4.30-4.70 (m, 9.8H), 5.59 (s, 2H), 6.16 (s, 2H), 7.07 (d, 4H), 8.07 (d, 4H)

### <Synthesis of 4-DPEGMAII>

A mixed liquid obtained by adding, to 12.8 g (0.09 mol) of glycidyl methacrylate, 12.9 g (0.05 mol) of 4,4'-diphenyletherdicarboxylic acid, 0.02 g (0.00009 mol) of tetrabutylammonium bromide, 0.02 g (0.00009 mol) of BHT, and 20 g of dimethylacetamide was subjected to a reaction at 100°C for 4 hours. To the liquid obtained by the reaction, 40 ml of ethyl acetate was added, and the mixture was turned into a homogeneous solution. Next, the solution was transferred to a separatory funnel and washed three times with 40 ml of a 10 wt% aqueous solution of potassium carbonate. The resultant was further washed three times with distilled water, and then an ethyl acetate layer was collected. After that, magnesium sulfate was added to the collected ethyl acetate layer to remove the water contained in the ethyl acetate layer. Subsequently, the magnesium sulfate was filtered off from the ethyl acetate layer, and the filtrate was concentrated with a rotary evaporator to provide a concentrate. The concentrate was further vacuum-dried to provide 4-DPEGMAII (yield: 25.3 g, percentage yield: 90%, HPLC purity: 95%). ¹H NMR spectrum data on the resultant 4-DPEGMAII was as follows.
¹H NMR δ 1.93 (s, 6H), 3.90 (d, 0.4H), 4.30-4.70 (m, 9.6H), 5.62 (s, 2H), 6.16 (s, 2H), 7.07 (d, 4H), 8.07 (d, 4H)

### <Synthesis of 4-DPEGMAIII>

4-DPEGMAII was synthesized by the above-mentioned method, and then purified by silica gel column chromatography (filler: SiO₂, developing solvent: ethyl acetate/hexane=3/1 to 2/1) to provide 4-DPEGMAIII (yield: 18.7 g, percentage yield: 69%, HPLC purity: 99%). ¹H NMR spectrum data on the resultant 4-DPEGMAIII was as follows.
¹H NMR δ 1.93 (s, 6H), 4.30-4.41 (m, 10H), 5.62 (s, 2H), 6.16 (s, 2H), 7.07 (d, 4H), 8.07 (d, 4H)

### <Synthesis of 4-DPEGMAIV>

4-DPEGMA was synthesized by the above-mentioned method, and then purified by silica gel column chromatography (filler: SiO₂, developing solvent: ethyl acetate/hexane=3/1 to 2/1) to provide 4-DPEGMAIV (yield: 2.2 g, percentage yield: 8%, HPLC purity: 99%) . ¹H NMR spectrum data on the resultant 4-DPEGMAIV was as follows. ¹H NMR δ 1.93 (s, 6H), 3.90 (d, 3.2H), 4.30-4.70 (m, 7.8H), 5.62 (s, 2H), 6.16 (s, 2H), 7.07 (d, 4H), 8.07 (d, 4H)

### <Synthesis of 4-DPEGA>

4-DPEGA (yield: 21.8 g, percentage yield: 85%, HPLC purity: 96%) was obtained by the same method as the synthesis method for 4-DPEGMA except that 11.5 g (0.09 mol) of glycidyl acrylate was used in place of 12.8 g (0.09 mol) of glycidyl methacrylate. ¹H NMR spectrum data on the resultant 4-DPEGA was as follows.
¹H NMR δ 1.93 (s, 6H), 3.84 (d, 2H), 4.00-4.65 (m, 8H), 5.83 (t, 2H), 6.12 (d, 2H), 6.43 (d, 2H), 7.09 (d, 4H), 7.99 (d, 4H)

### <Synthesis of 2-DPEGMA>

2-DPEGMA (yield: 23.0 g, percentage yield: 85%, HPLC purity: 95%) was obtained by the same method as the synthesis method for 4-DPEGMA except that 12.9 g (0.05 mol) of 2,2'-diphenyletherdicarboxylic acid was used in place of 12.9 g (0.05 mol) of 4,4'-diphenyletherdicarboxylic acid. ¹H NMR spectrum data on the resultant 2-DPEGMA was as follows.
¹H NMR δ 1.93 (s, 6H), 3.80 (d, 1.8H), 4.00-4.65 (m, 8.2H), 5.59 (s, 2H), 6.16 (s, 2H), 7.07 (d, 2H), 7.42 (d, 4H), 8.17 (d, 2H)

### <Synthesis of 4-DPEHGMA>

A solution was prepared by dissolving 5-hexen-1-ol (30.1 g, 0.3 mol) in 100 ml of methylene chloride and then adding 33.4 g (0.33 mol) of triethylamine and 1.8 g (0.015 mol) of N,N-dimethylaminopyridine, and then the solution was cooled with ice. Next, to the solution cooled with ice, a methylene chloride solution obtained by dissolving 31.4 g (0.3 mol) of methacrylic acid chloride in methylene chloride (50 ml) was added dropwise. After the completion of the dropwise addition, the resultant solution was stirred at room temperature for 3 hours, and then 100 ml of distilled water was added, followed by three times of extraction with methylene chloride . The solvent was removed from the methylene chloride layer obtained by the extraction through the use of a rotary evaporator to provide a residue. Further, the resultant residue was dissolved in 100 ml of toluene. The resultant toluene solution was washed three times with a 0.5 N hydrochloric acid solution, then washed three times with brine, and dried over magnesium sulfate . After the drying, the magnesium sulfate was filtered off, and the filtrate was concentrated with a rotary evaporator. The concentrate was further vacuum-dried to provide 46.4 g of 5-hexen-1-yl methacrylate (percentage yield: 92%).

45.4 g (0.27 mol) of the resultant 5-hexen-1-yl methacrylate was dissolved in 100 ml of methylene chloride, and then 196 g of a 60 mass% m-chloroperbenzoic acid/water mixture (corresponding to 0.675 mol) was added, followed by stirring at room temperature for 10 hours. After the stirring, m-chlorobenzoic acid, a by-product, was filtered off, and the filtrate was subjected to reducing treatment with 150 ml of a 15 mass% aqueous solution of sodium sulfite. The methylene chloride layer separated from the filtrate subjected to the reducing treatment was washed twice with a 5 mass% aqueous solution of potassium carbonate, dried over magnesium sulfate, and then concentrated with a rotary evaporator to provide a concentrate. Then, the concentrate was further vacuum-dried to provide 44.8 g of 5,6-epoxyhexan-1-yl methacrylate (percentage yield: 90%).

A mixed liquid obtained by mixing 23.0 g (0.125 mol) of the resultant 5,6-epoxyhexan-1-yl methacrylate, 12.9 g (0.05 mol) of 4,4'-diphenyletherdicarboxylic acid, 0.045 g (0.2 mmol) of benzyltriethylammonium chloride, and 0.03 g of p-methoxyphenol was stirred at 90°C for 4 hours. After the heating and stirring, the mixed liquid was allowed to cool to room temperature, and then 50 ml of water was added, followed by extraction with methylene chloride. The resultant methylene chloride layer was dried over magnesium sulfate, and then concentrated with a rotary evaporator to provide a concentrate. Further, the concentrate was vacuum-dried to provide 4-DPEHGMA (yield: 29.0 g, 0.046 mol, percentage yield: 93%, HPLC purity: 95%). ¹H NMR spectrum data on the resultant 4-DPEHGMA was as follows.
¹H NMR δ 1.29 (t, 4H), 1.44 (m, 4H), 1.57 (t, 4H), 1.93 (s, 6H), 3.85 (d, 1.9H), 4.15-4.70 (m, 8.1H), 5.59 (s, 2H), 6.16 (s, 2H), 7.05 (d, 4H), 7.95 (d, 4H)

### <Synthesis of 4-DPEEGMA>

A solution was prepared by dissolving 30.6 g (0.3 mol) of allyloxyethanol in 100 ml of methylene chloride and then further adding 33.4 g (0.33 mol) of triethylamine and 1.8 g (0.015 mol) of N,N-dimethylaminopyridine. Next, the resultant solution was cooled with ice, and to the solution, a solution obtained by dissolving 31.4 g (0.3 mol) of methacrylic acid chloride in 50 ml of methylene chloride was added dropwise. After the completion of the dropwise addition, the resultant solution was stirred at room temperature for 3 hours, and then 100 ml of distilled water was added, followed by three times of extraction with methylene chloride. Next, a residue obtained by removing the solvent from the resultant methylene chloride layer using a rotary evaporator was dissolved in 100 ml of toluene to provide a toluene solution. Then, the toluene solution was washed three times with a 0.5 N hydrochloric acid solution, then washed three times with brine, and dried over magnesium sulfate . After the drying, the magnesium sulfate was filtered off, and the filtrate was concentrated with a rotary evaporator to provide a concentrate. Further, the concentrate was vacuum-dried to provide 46.0 g of allyloxyethyl methacrylate (percentage yield: 90%).

A solution prepared by dissolving 46.0 g (0.27 mol) of the resultant allyloxyethyl methacrylate in 100 ml of methylene chloride and further adding 196 g of a 60 mass% m-chloroperbenzoic acid/water mixture (corresponding to 0.675 mol) was stirred at room temperature for 10 hours. m-Chlorobenzoic acid, a by-product, was filtered off from the solution after the stirring, and the filtrate was subjected to reducing treatment with 150 ml of a 15 mass% aqueous solution of sodium sulfite. Subsequently, the methylene chloride layer separated from the filtrate subjected to the reducing treatment was washed twice with a 5 mass% aqueous solution of potassium carbonate, dried over magnesium sulfate, and then concentrated with a rotary evaporator to provide a concentrate. Further, the concentrate was vacuum-dried to provide 47.3 g of glycidyloxyethyl methacrylate (percentage yield: 94%).

A mixed liquid obtained by mixing 23.3 g (0.125 mol) of the resultant glycidyloxyethyl methacrylate, 12.9 g (0.05 mol) of 4,4'-diphenyletherdicarboxylic acid, 0.045 g (0.2 mmol) of benzyltriethylammonium chloride, and 0.03 g of p-methoxyphenol was stirred at 90°C for 4 hours. After the heating and stirring, the mixed liquid was allowed to cool to room temperature, and 50 ml of water was added, followed by extraction with methylene chloride. The resultant methylene chloride layer was dried over magnesium sulfate, and then concentrated with a rotary evaporator to provide a concentrate. Finally, the concentrate was vacuum-dried to provide 4-DPEEGMA (yield: 29.0 g, percentage yield: 92%, HPLC purity: 94%). ¹H NMR spectrum data on the resultant 4-DPEEGMA was as follows. ¹HNMR δ 1.93 (s, 6H), 3.50-4.50 (m, 18H), 4.15-4.70 (m, 8.1H), 5.59 (s, 2H), 6.16 (s, 2H), 7.02 (d, 4H), 8.02 (d, 4H)

### <Synthesis of 4-DPSGMA (for reference only, not according to the invention)>

48.4 g (0.2 mol) of 4,4'-diformyldiphenyl sulfide synthesized by a synthesis method disclosed in JP 2005-154379 A was dissolved in 200 ml of t-butanol and 50 ml of water. After that, 50 ml of an aqueous solution of sodium hydrogen phosphate and 140 g (2 mol) of 2-methyl-2-butene were added, and 36 g (0.4 mol) of sodium chlorite was further added to prepare a reaction solution. Next, the reaction solution was stirred for 5 hours, and then the reaction solution was rendered acidic using a 1 N hydrochloric acid solution to precipitate a solid. Subsequently, the reaction solution having the solid precipitated therein was subjected to suction filtration, and then the precipitated solid was washed with water. After the washing, the resultant solid (compound) was vacuum-dried to provide 45.5 g of 4,4'-dicarboxydiphenyl sulfide (percentage yield: 83%) .

Then, 4-DPSGMA (yield: 22.3 g, percentage yield: 80%, HPLC purity: 95%) was obtained by the same method as the synthesis method for 4-DPEGMA except that 13.7 g (0.05 mol) of the above-mentioned 4,4'-dicarboxydiphenyl sulfide was used in place of 12.9 g of 4,4'-diphenyletherdicarboxylic acid. ¹H NMR spectrum data on the resultant 4-DPSGMA was as follows.
¹H NMR δ 1.93 (s, 6H), 3.62 (d, 2H), 3.90-4.70 (m, 8H), 5.58 (s, 2H), 6.14 (s, 2H), 7.33 (d, 4H), 7.78 (d, 4H)

### <Synthesis of 4-DPSOGMA (for reference only, not according to the invention)>

4-DPSOGMA (yield: 24.5 g, percentage yield: 83%, HPLC purity: 97%) was obtained by the same method as the synthesis method for 4-DPEGMA except that 15.3 g (0.05 mol) of 4,4'-dicarboxydiphenyl sulfone was used in place of 12.9 g of 4,4'-diphenyletherdicarboxylic acid. ¹H NMR spectrum data on the resultant 4-DPSOGMA was as follows. ¹H NMR δ 1.93 (s, 6H), 3.70 (d, 2H), 3.90-4.70 (m, 8H), 5.58 (s, 2H), 6.14 (s, 2H), 8.04 (d, 4H), 8.20 (d, 4H)

### <Synthesis of 4-DPFGMA (for reference only, not according to the invention)>

44.8 g (0.2 mol) of 4,4'-diformyldiphenylmethane synthesized by a synthesis method disclosed in JP 2005-154379 A was dissolved in 200 ml of t-butanol and 50 ml of water. After that, 50 ml of an aqueous solution of sodium hydrogen phosphate and 140 g (2 mol) of 2-methyl-2-butene were added, and 36 g (0.4 mol) of sodium chlorite was further added to prepare a reaction solution. Next, the reaction solution was stirred for 5 hours, and then the reaction solution was rendered acidic using a 1 N hydrochloric acid solution to precipitate a solid. Subsequently, the reaction solution having the solid precipitated therein was subjected to suction filtration, and then the precipitated solid was washed with water. After the washing, the resultant solid (compound) was vacuum-dried to provide 39.8 g of 4,4'-dicarboxydiphenylmethane (percentage yield: 78%).

Then, 4-DPFGMA (yield: 22.1 g, percentage yield: 82%, HPLC purity: 94%) was obtained by the same method as the synthesis method for the 4-DPEGMA except that 12.8 g (0.05 mol) of 4,4'-dicarboxydiphenylmethane obtained above was used in place of 12.9 g of 4,4'-diphenyletherdicarboxylic acid. ¹H NMR spectrum data on the resultant 4-DPFGMA was as follows.
¹H NMR δ 1.93 (s, 6H), 3.78 (s, 2H), 3.90 (d, 2H), 4.00-4.70 (m, 8H), 5.58 (s, 2H), 6.14 (s, 2H), 7.20 (d, 4H), 7.87 (d, 4H)

### <Synthesis of 4-DPAGMA (for reference only, not according to the invention)>

4-DPAGMA (yield: 22.7 g, percentage yield: 80%, HPLC purity: 93%) was obtained by the same method as the synthesis method for the 4-DPEGMA except that 14.2g (0.05 mol) of 2,2-bis(4-carboxyphenyl)propane synthesized by a synthesis method disclosed in GB 753384 A was used in place of 12.9 g of 4,4'-diphenyletherdicarboxylic acid. ¹H NMR spectrum data on the resultant 4-DPAGMA was as follows.
¹H NMR δ 1.65 (s, 6H), 1.93 (s, 6H), 3.85 (d, 2H), 3.90-4.65 (m, 8H), 5.57 (s, 2H), 6.12 (s, 2H), 7.23 (d, 4H), 7.90 (d, 4H)

### <Synthesis of 4-DPBGMA (for reference only, not according to the invention)>

50.8 g (0.2 mol) of 4-formylphenyl-4'-formylbenzoate synthesized by a synthesis method disclosed in JP 2007-106779 A was dissolved in 200 ml of t-butanol and 50 ml of water. After that, 50 ml of an aqueous solution of sodium hydrogen phosphate and 140 g (2 mol) of 2-methyl-2-butene were added, and 36 g (0.4 mol) of sodium chlorite was further added to prepare a reaction solution. Next, the reaction solution was stirred for 5 hours, and then the reaction solution was rendered acidic using a 1 N hydrochloric acid solution to precipitate a solid. Subsequently, the reaction solution having the solid precipitated therein was subjected to suction filtration, and then the precipitated solid was washed with water. After the washing, the resultant solid (compound) was vacuum-dried to provide 45.4 g of 4-carboxyphenyl-4'-carboxybenzoate (percentage yield: 79%).

Then, 4-DPBGMA (yield: 22.8 g, percentage yield: 80%, HPLC purity: 94%) was obtained by the same method as the synthesis method for 4-DPEGMA except that 14.3 g (0.05 mol) of the above-mentioned 4-carboxyphenyl-4'-carboxybenzoate was used in place of 12.9 g of 4,4'-diphenyletherdicarboxylic acid. ¹H NMR spectrum data on the resultant 4-DPBGMA was as follows.
¹H NMR δ 1.93 (s, 6H), 3.87 (d, 2H), 3.90-4.65 (m, 8H), 5.60 (s, 2H), 6.14 (s, 2H), 7.25 (d, 2H), 8.10 (d, 2H), 8.15 (d, 2H), 8.27 (d, 2H)

### <Synthesis of 4-DPALGMA (for reference only, not according to the invention)>

1,1-Bis(4-formylphenyl)cyclohexane (58.4 g, 0.2 mol) synthesized by a synthesis method disclosed in JP 3076603 B2 was dissolved in 200 ml of t-butanol and 50 ml of water. After that, 50 ml of an aqueous solution of sodium hydrogen phosphate and 140 g (2 mol) of 2-methyl-2-butene were added, and 36 g (0.4 mol) of sodium chlorite was further added to prepare a reaction solution. Next, the reaction solution was stirred for 5 hours, and then the reaction solution was rendered acidic using a 1 N hydrochloric acid solution to precipitate a solid. Subsequently, the reaction solution having the solid precipitated therein was subjected to suction filtration, and then the precipitated solid was washed with water. After the washing, the resultant solid (compound) was vacuum-dried to provide 54.4 g of 1,1-bis(4-carboxylphenyl)cyclohexane (percentage yield: 84%).

Then, 4-DPALGMA (yield: 23.7g, percentage yield: 78%, HPLC purity: 92%) was obtained by the same method as the synthesis method for 4-DPEGMA except that 16.2 g (0.05 mol) of 1,1-bis(4-carboxylphenyl)cyclohexane was used in place of 12.9 g of 4,4'-diphenyletherdicarboxylic acid. ¹H NMR spectrum data on the resultant 4-DPALGMA was as follows.
¹H NMR δ 1.42 (t, 6H), 1.93 (s, 6H), 2.10 (t, 4H), 3.77 (d, 2H), 3.85-4.60 (m, 8H), 5.61 (s, 2H), 6.15 (s, 2H), 7.24 (d, 4H), 7.90 (d, 4H)

### (7) Evaluation Results

The compositions of the curable composition samples prepared by using the first polymerizable monomers and the evaluation results of the bending strengths of their cured products are shown in Tables 1 to 3, and the compositions of the curable composition samples prepared using the first polymerizable monomers and the evaluation results of their adhesive strengths are shown in Table 4.

**[Table 1]**

| | Composition of curable composition sample (part(s) by mass) | | | | | Bending strength/MPa (deviation) |
|---|---|---|---|---|---|---|
| | First polymerizable monomer (60) | | Second polymerizable monomer (40) | Other components, such as polymerization initiator | Filler | |
| | Abbreviation | Viscosity/P a·s | | | | |
| Example A1 | 4-DPEHE | 0.75 | 3G | | - | 60 (3.7) |
| Example A2 | | | | | | 149 (7.1) |
| Example A3 | | | ND | | | 147 (5.9) |
| Example A4 | | | TCD | | | 138 (6.2) |
| Example A5 | 2-DPEHE | 0.75 | 3G | | | 147 (7.0) |
| Example A6 | 4-DPEHP | 0.65 | | | | 149 (7.8) |
| Example A7 | 4-DPEHB | 0.60 | | | | 145 (6.5) |
| Example A8 | 4-DPEHH | 0.50 | | | | 138 (6.3) |
| Example A9 | 4-DPEHIP | 0.65 | | | | 144 (6.8) |
| Example A10 | 4-DPEPE | 0.68 | | | | 140 (6.6) |
| Example A11 | 4-DPEGAM | 0.88 | | | | 138 (6.9) |
| Example A12 | 4-DPEU | 0.80 | | | | 140 (6.4) |
| Example A13 | 4-DPEUE | 0.78 | | | | 136 (6.3) |
| Example A14 (for reference only) | 4-DPSHE | 0.79 | | CQ (0.5)/DMBE (0.8)/BHT (0.1) | F1 (205.9) | 139 (5.7) |
| Example A15 (for reference only) | 4-DPSOHE | 0.81 | | | | 140 (6.1) |
| Example A16 (for reference only) | 4-DPFHE | 0.78 | | | | 143 (5.8) |
| Example A17 (for reference | 4-DPAHE | 0.82 | | | | 141 (6.6) |
| only) | | | | | | |
| Example A18 (for reference only) | 4-DPBHE | 0.76 | | | | 135 (6.0) |
| Example A19 (for reference only) | 4-DPALHE | 0.78 | | | | 134 (6.2) |

**[Table 2]**

| | Composition of curable composition sample (part(s) by mass) | | | | | Bending strength/MPa (deviation) | |
|---|---|---|---|---|---|---|---|
| | First polymerizable monomer (60) | | Second polymerizable monomer (40) | Other components, such as polymerization initiator | Filler | | |
| | Abbreviation | Viscosity/ Pa·s | | | | Initial | After storage at 60°C |
| Example A20 | 4-DPEGMA | 11 | 3G | | - | 65 (2.1) | - |
| Example A21 | | | | | | 157 (7.1) | 143 (3.3) |
| Example A22 | | | ND | | | 155 (3.9) | - |
| Example A23 | | | TCD | | | 148 (6.4) | - |
| Example A24 | 4-DPEGMAI | 11 | 3G | | | 158 (6.9) | 145 (4.5) |
| Example A25 | 4-DPEGMAII | 11 | | | | 158 (8.2) | 150 (2.7) |
| Example A26 | 4-DPEGMAIII | 11 | | | | 162 (5.3) | 115 (2.9) |
| Example A27 | 4-DPEGMAIV | 11 | | | | 151 (3.2) | 148 (2.3) |
| Example A28 | 4-DPEGA | 11 | | | | 157 (7.3) | - |
| Example A29 | 2-DPEGMA | 12 | | | | 153 (6.8) | - |
| Example A30 | 4-DPEHGMA | 7.3 | | | | 142 (4.5) | - |
| Example A31 | 4-DPEEGMA | 6.3 | | | | 141 (4.8) | - |
| Example A32 (for reference only) | 4-DPSGMA | 17 | | CQ (0.5)/DMBE (0.8)/BHT (0.1) | F1 (205.9) | 150 (4.8) | - |
| Example A33 (for reference only) | 4-DPSOGMA | 19 | | | | 146 (6.1) | - |
| Example A34 (for reference only) | 4-DPFGMA | 14 | | | | 154 (3.9) | - |
| Example A35 (for reference only) | 4-DPAGMA | 20 | | | | 148 (4.4) | - |
| Example A36 (for reference only) | 4-DPBGMA | 19 | | | | 143 (4.2) | - |
| Example A37 (for reference only) | 4-DPALGMA | 18 | | | | 142 (4.4) | - |

**[Table 3]**

| | Composition of curable composition sample (part(s) by mass) | | | | | Bending strength/MPa (deviation) |
|---|---|---|---|---|---|---|
| | First polymerizable monomer (60) | | Second polymerizable monomer (40) | Other components, such as polymerization initiator | Filler | |
| | Abbreviation | Viscosity/Pa· s | | | | |
| Comparative Example A1 | 4-BPDM | Unmeasurable (solid) | 3G | CQ (0.5)/DMBE (0.8)/BHT (0.1) | F1 (205.9) | 108 (6.7) |
| Comparative Example A2 | 4-BPGD | Unmeasurable (solid) | | | | 105 (6.8) |
| Comparative Example A3 | 4-DPHU | Unmeasurable (solid) | | | | 101 (4.3) |
| Comparative Example A4 | 4-BPEHE | Unmeasurable (solid) | | | | 110 (5.5) |
| Comparative Example A5 | Bis-GMA | >100 | | | - | 60 (2.3) |
| Comparative Example A6 | Bis-GMA | >100 | | | F1 (205.9) | 138 (7.2) |

**[Table 4]**

| | Composition of curable composition sample (part(s) by mass) | | | Adhesive strength/MPa (deviation) | | |
|---|---|---|---|---|---|---|
| | First polymerizable monomer (30) | Second polymerizable monomer | Other components, such as polymerization initiator | | | |
| | | | | Initial | After endurance test | After storage at 60°C |
| Example B1 | 4-DPEGMA | 3G (20)/PM (30) /HEMA (20) | CQ (1.5)/DMBE (1.5)BHT (0.3)/water (10)/acetone (85) | 13.5 (1.2) | 12.7 (1.0) | 11.2 (1.9) |
| Example B2 | 4-DPEGMAI | | | 13.9 (1.3) | 12.9 (1.3) | 10.7 (2.2) |
| Example B3 | 4-DPEGMAII | | | 13.6 (0.9) | 12.7 (1.5) | 10.9 (1.2) |
| Example B4 | 4-DPEGMAIII | | | 14.0 (1.1) | 12.8 (1.6) | 6.2 (1.4) |
| Example B5 | 4-DPEGMAIV | | | 12.7 (1.6) | 12.2 (1.2) | 12.2 (1.9) |
| Example B6 | 4-DPEGA | | | 13.7 (1.2) | 12.8 (1.1) | - |
| Example B7 | 2-DPEGMA | | | 13.3 (0.9) | 12.4 (1.1) | - |
| Example B8 | 4-DPEHGMA | | | 10.6 (1.6) | 9.1 (0.9) | - |
| Example B9 | 4-DPEEGMA | | | 12.6 (1.3) | 11.8 (1.1) | - |
| Example B10 (for reference only) | 4-DPSGMA | | | 11.1 (1.7) | 9.3 (1.6) | - |
| Example B11 (for reference only) | 4-DPSOGMA | | | 10.8 (1.1) | 8.9 (0.8) | - |
| Example B12 (for reference only) | 4-DPFGMA | | | 11.5 (1.3) | 10.8 (0.9) | - |
| Example B13 (for reference only) | 4-DPAGMA | | | 10.1 (1.7) | 8.7 (0.8) | - |
| Example B14 (for reference only) | 4-DPBGMA | | | 11.7 (1.1) | 9.8 (0.8) | - |
| Example B15 (for reference only) | 4-DPALGMA | | | 10.2 (1.6) | 8.7 (0.9) | - |
| Comparative Example B1 | Bis-GMA | | | 5.8 (1.0) | 4.8 (0.7) | - |

## Claims

1. A polymerizable monomer, which is represented by the following general formula (1): 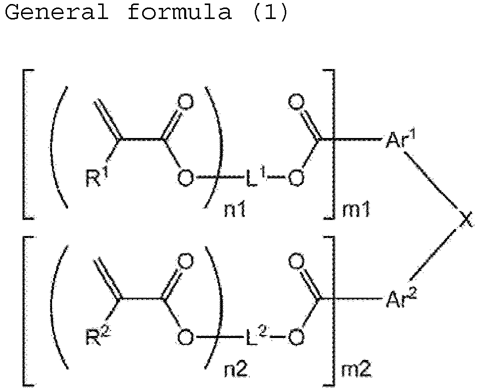 in the general formula (1), X represents a divalent group, Ar¹ and Ar² each represent an unsubstituted aromatic group having a valence selected from divalence to tetravalence, and may be identical to or different from each other, L¹ and L² each represent a substituted or unsubstituted hydrocarbon group having a main chain with a number of atoms within a range of from 2 to 60 and having a valence selected from divalence to tetravalence, and may be identical to or different from each other, R¹ and R² each represent a hydrogen atom or a methyl group, and m1, m2, n1, and n2 each represent an integer selected from a range of from 1 to 3,
wherein the divalent group X is -O-.

2. A polymerizable monomer according to claim 1, wherein the carbon atoms constituting a main chain of the hydrocarbon group may be substituted by a heteroatom, preferably wherein the heteroatom is an oxygen atom, a nitrogen atom, a sulfur atom, or a silicon atom.

3. A polymerizable monomer according to claim 1 or 2, wherein the atoms constituting the main chain of the hydrocarbon group may have bonded thereto a substituent, preferably wherein the substituent is an alkyl group having 1 to 3 carbon atoms, a hydroxy group, a monovalent hydrocarbon group having a hydroxy group, a halogen atom, -COOR⁴, or -OR⁴, wherein R⁴ is the same as the alkyl group having 1 to 3 carbon atoms.

4. A polymerizable monomer according to any one of claims 1 to 3, wherein at least any one of the hydrocarbon groups L¹ and L², each of which has a main chain with a number of atoms within a range of from 2 to 60 and has a valence selected from divalence to tetravalence, contains a hydroxy group.

5. A polymerizable monomer according to any one of claims 1 to 4, wherein the polymerizable monomer is represented by the following general formula (2): 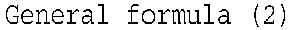 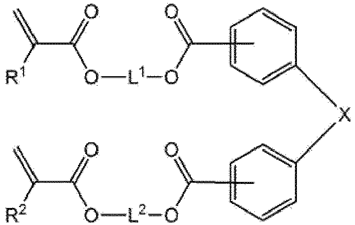 in the general formula (2), X, L¹, L², R¹, and R² are the same as those shown in the general formula (1).

6. A polymerizable monomer according to any one of claims 1 to 4, wherein the polymerizable monomer is represented by the following general formula (3): 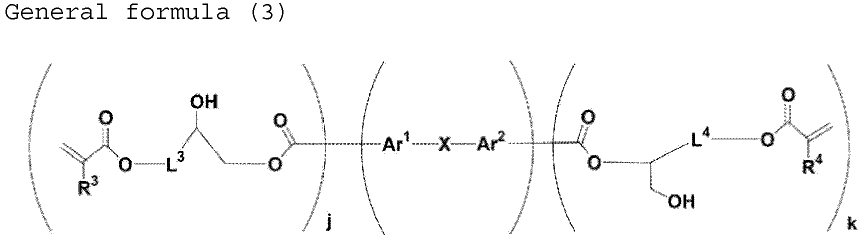 in the general formula (3), X is the same as that shown in the general formula (1), Ar¹ and Ar² are the same as those shown in the general formula (1) except that the valence may be only divalence, L³ and L⁴ each represent a divalent hydrocarbon group having a main chain with a number of atoms within a range of from 1 to 8, and may be identical to or different from each other, R³ and R⁴ each represent a hydrogen atom or a methyl group, j represents 0, 1, or 2, k represents 0, 1, or 2, and j+k=2.

7. A polymerizable monomer according to claim 6, wherein a combination (j, k) of values j and k shown in the general formula (3) comprises a combination selected from the group consisting of (1, 1) and (0, 2).

8. A polymerizable monomer according to claim 6, wherein the polymerizable monomer comprises two or more kinds of structural isomers in each of which a combination (j, k) of values j and k shown in the general formula (3) is selected from the group consisting of (2, 0), (1, 1), and (0, 2).

9. A polymerizable monomer according to claim 8, wherein an average value of values k in all molecules of the polymerizable monomer is 0.05 or more and less than 2.0.

10. A method of producing a polymerizable monomer, the polymerizable monomer comprising two or more kinds of structural isomers selected from the group consisting of compounds represented by the following general formulae (6) to (8), the method comprising at least a reaction step of allowing a compound represented by the following general formula (4) and a compound represented by the following general formula (5) to react with each other: 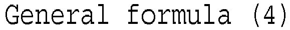 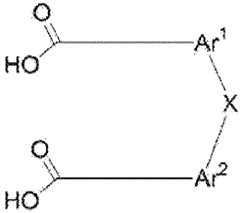 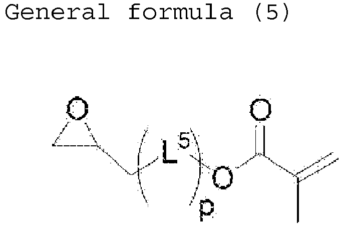 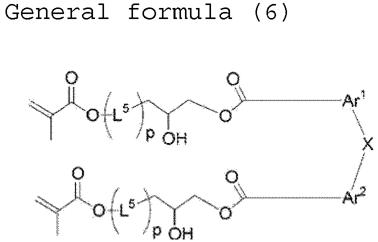 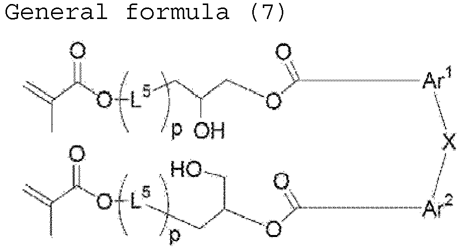 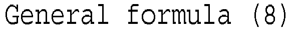 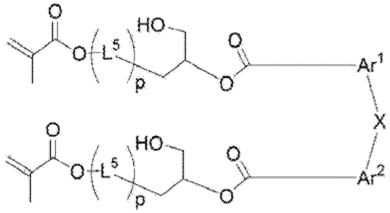 in the general formulae (4) to (8), X represents a divalent group, Ar¹ and Ar² each represent a divalent and unsubstituted aromatic group, and may be identical to or different from each other, L⁵ represents a divalent hydrocarbon group having a main chain with a number of atoms of from 1 to 7, and p represents 0 or 1, wherein the divalent group X is -O-.

11. A curable composition, comprising:
the polymerizable monomer of any one of claims 1 to 9; and
a polymerization initiator.

12. A resin member, comprising a cured product obtained by using a composition containing the polymerizable monomer of any one of claims 1 to 9.

## Patentansprüche

1. Ein polymerisierbares Monomer, das durch die folgende allgemeine Formel (1) repräsentiert wird: 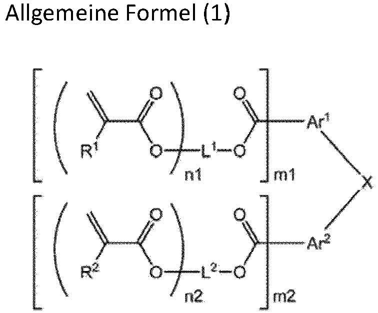 in der allgemeinen Formel (1) repräsentiert X eine divalente Gruppe, Ar¹ und Ar² repräsentieren jeweils eine unsubstituierte aromatische Gruppe mit einer Valenz, die aus Divalenz bis Tetravalenz ausgewählt wird, und können identisch oder verschieden voneinander sein, L¹ und L² repräsentieren jeweils eine substituierte oder unsubstituierte Kohlenwasserstoffgruppe mit einer Hauptkette mit einer Anzahl von Atomen in einem Bereich von 2 bis 60 und einer Valenz, due aus Divalenz bis Tetravalenz ausgewählt wird, und können identisch oder verschieden voneinander sein; R¹ und R² repräsentieren jeweils ein Wasserstoffatom oder eine Methylgruppe und m1, m2, n1 und n2 repräsentieren jeweils eine Ganzzahl, die aus einem Bereich von 1 bis 3 ausgewählt wird,
wobei die divalente Gruppe X -O- ist.

2. Ein polymerisierbares Monomer entsprechend Anspruch 1, wobei die Kohlenstoffatome, die eine Hauptkette der Kohlenwasserstoffgruppe ausmachen, durch ein Heteroatom substituiert werden können, wobei das Heteroatom bevorzugt ein Sauerstoffatom, ein Stickstoffatom, ein Schwefelatom oder ein Siliziumatom ist.

3. Ein polymerisierbares Monomer entsprechend Anspruch 1 oder 2, wobei die Kohlenstoffatome, welche die Hauptkette der Kohlenwasserstoffgruppe ausmachen, dieser einen Substituent gebunden haben können, wobei der Substituent bevorzugt eine Alkylgruppe mit 1 bis 3 Kohlenstoffatomen, eine Hydroxygruppe, eine monovalente Kohlenwasserstoffgruppe mit einer Hydroxygruppe, ein Halogenatom, -COOR⁴ oder -OR⁴ ist, wobei R⁴ dieselbe wie die Alkylgruppe mit 1 bis 3 Kohlenstoffatomen ist.

4. Ein polymerisierbares Monomer entsprechend einem der Ansprüche 1 bis 3, wobei zumindest eine der Kohlenwasserstoffgruppen L¹ und L², die jeweils eine Hauptkette mit einer Anzahl von Atomen in einem Bereich von 2 bis 60 haben und eine Valenz haben, die aus Divalenz bis Tetravalenz ausgewählt wird, eine Hydroxygruppe enthalten.

5. Ein polymerisierbares Monomer entsprechend einem der Ansprüche 1 bis 4, wobei das polymerisierbare Monomer durch die allgemeine Formel (2) repräsentiert wird: 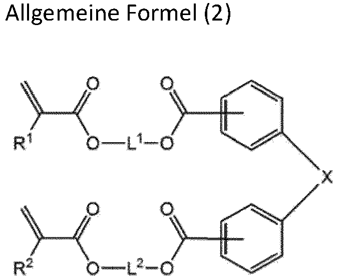 in der allgemeinen Formel (2) sind X, L¹, L², R¹ und R² dieselben wie diejenigen, die in der allgemeinen Formel (1) gezeigt werden.

6. Ein polymerisierbares Monomer entsprechend einem der Ansprüche 1 bis 4, wobei das polymerisierbare Monomer durch die allgemeine Formel (3) repräsentiert wird: 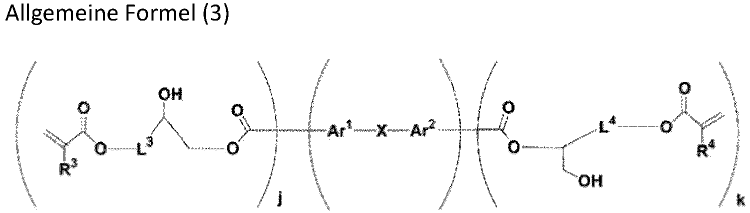 in der allgemeinen Formel (3) ist X dasselbe wie das, das in der allgemeinen Formel (1) angezeigt wird, Ar¹ und Ar² sind dieselben wie diejenigen, die sie in der allgemeinen Formel (1) angezeigt werden, mit Ausnahme, dass die Valenz nur Divalenz sein kann, L³ und L⁴ repräsentieren jeweils eine divalente Kohlenwasserstoffgruppe mit einer Hauptkette mit einer Anzahl von Atomen in einem Bereich von 1 bis 8 und können identisch oder verschieden voneinander sein, R³ und R⁴ repräsentieren jeweils ein Wasserstoffatom oder eine Methylgruppe, j repräsentiert 0, 1 oder 2, k repräsentiert 0, 1, oder 2 und j+k = 2.

7. Ein polymerisierbares Monomer entsprechend Anspruch 6, wobei eine Kombination (j, k) der Werte j und k, die in der allgemeinen Formel (3) gezeigt werden, eine Kombination umfasst, die aus der Gruppe bestehend aus (1, 1) und (0, 2) ausgewählt wird.

8. Ein polymerisierbares Monomer entsprechend Anspruch 6, wobei das polymerisierbare Monomer zwei oder mehr Arten von Strukturisomeren umfasst, wobei in jedem dieser eine Kombination (j, k) der Werte j und k, die in der allgemeinen Formel (3) gezeigt werden, aus der Gruppe bestehen aus (2, 0), (1, 1) und (0, 2) ausgewählt wird.

9. Ein polymerisierbares Monomer entsprechend Anspruch 8, wobei der Durchschnittswert der Werte k in allen Molekülen des polymerisierbaren Monomers 0,05 oder mehr und weniger als 2,0 beträgt.

10. Ein Verfahren zur Erzeugung eines polymerisierbaren Monomers, wobei das polymerisierbare Monomer eine oder mehr Arten von Strukturisomeren umfasst, die aus Gruppen ausgewählt werden, die aus Verbindungen bestehen, die durch die folgenden Formeln (6) bis (8) repräsentiert werden, wobei das Verfahren zumindest einen Reaktionsschritt umfasst, der erlaubt, dass eine Verbindung, die durch die folgende allgemeine, Formel (4) repräsentiert wird, und eine Verbindung, die durch die folgende allgemeine Formel (5) repräsentiert wird, miteinander reagieren: 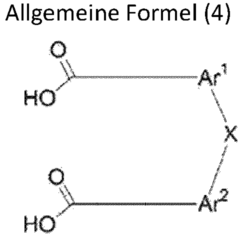 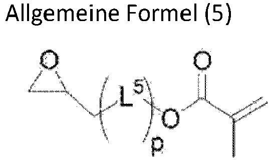 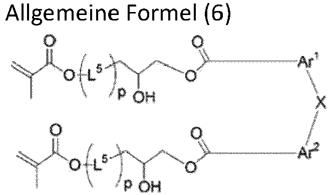 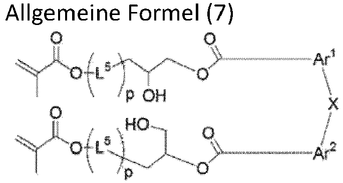 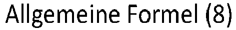 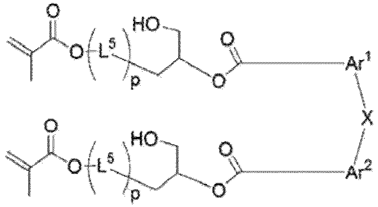 in den allgemeinen Formeln (4) bis (8) repräsentiert X eine divalente Gruppe, Ar¹ und Ar² repräsentieren jeweils eine divalente und unsubstituierte aromatische Gruppe und können identisch oder verschieden voneinander sein, L⁵ repräsentiert eine divalente Kohlenwasserstoffgruppe mit einer Hauptkette mit einer Anzahl von Atomen von 1 bis 7 und p repräsentiert 0 oder 1, wobei die divalente Gruppe X -O- ist.

11. Eine härtbare Zusammensetzung, die Folgendes umfasst:
das polymerisierbare Monomer von einem der Ansprüche 1 bis 9 und
einen Polymerisationsinitiator.

12. Ein Harzelement, das ein gehärtetes Produkt umfasst, das durch Verwendung einer Zusammensetzung, welche das polymerisierbare Monomer von einem der Ansprüche 1 bis 9 enthält, erhalten wird.

## Revendications

1. Monomère polymérisable, qui est représenté par la formule générale (1) suivante : 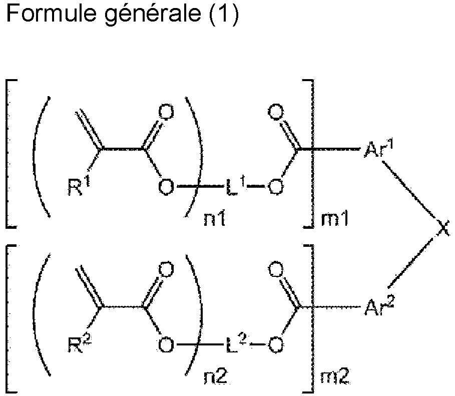 dans la formule générale (1), X représente un groupe divalent, Ar¹ et Ar² représentent chacun un groupe aromatique non substitué ayant une valence sélectionnée entre la divalence et la tétravalence, et peuvent être identiques ou différents l'un de l'autre, L¹ et L² représentent chacun un groupe hydrocarboné substitué ou non substitué ayant une chaîne principale avec un nombre d'atomes compris entre 2 et 60 et ayant une valence sélectionnée entre la divalence et la tétravalence, et peuvent être identiques ou différents l'un de l'autre, R¹ et R² représentent chacun un atome d'hydrogène ou un groupe méthyle, et m1, m2, n1 et n2 représentent chacun un nombre entier sélectionné dans une plage comprise entre 1 et 3,
dans laquelle formule le groupe divalent X représente -O-.

2. Monomère polymérisable selon la revendication 1, dans lequel les atomes de carbone constituant une chaîne principale du groupe hydrocarboné peuvent être substitués par un hétéroatome, de préférence dans lequel l'hétéroatome est un atome d'oxygène, un atome d'azote, un atome de soufre ou un atome de silicium.

3. Monomère polymérisable selon la revendication 1 ou la revendication 2, dans lequel les atomes constituant la chaîne principale du groupe hydrocarboné peuvent avoir un substituant qui leur est lié, de préférence dans lequel le substituant est un groupe alkyle portant 1 à 3 atomes de carbone, un groupe hydroxy, un groupe hydrocarboné monovalent ayant un groupe hydroxy, un atome d'halogène, -COOR⁴ ou -OR⁴, où R⁴ est identique au groupe alkyle portant 1 à 3 atomes de carbone.

4. Monomère polymérisable selon l'une quelconque des revendications 1 à 3, dans lequel au moins l'un quelconque des groupes hydrocarbonés L¹ et L², dont chacun a une chaîne principale avec un nombre d'atomes compris dans une plage de 2 à 60 et a une valence sélectionnée entre la divalence et la tétravalence, contient un groupe hydroxy.

5. Monomère polymérisable selon l'une quelconque des revendications 1 à 4, dans lequel le monomère polymérisable est représenté par la formule générale (2) suivante : 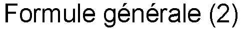 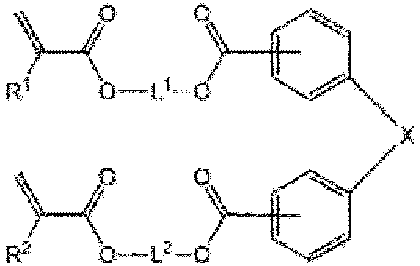 dans la formule générale (2), X, L¹, L², R¹ et R² sont des éléments identiques à ceux indiqués dans la formule générale (1).

6. Monomère polymérisable selon l'une quelconque des revendications 1 à 4, dans lequel le monomère polymérisable est représenté par la formule générale (3) suivante : 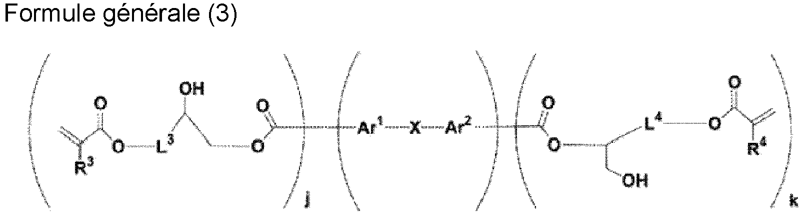 dans la formule générale (3), X est un élément identique à celui indiqué dans la formule générale (1), Ar¹ et Ar² sont des éléments identiques à ceux indiqués dans la formule générale (1) sauf que la valence peut être seulement une divalence, L³ et L⁴ représentent chacun un groupe hydrocarboné divalent ayant une chaîne principale avec un nombre d'atomes compris dans la plage allant de 1 à 8, et peuvent être identiques ou différents l'un de l'autre, R³ et R⁴ représentent chacun un atome d'hydrogène ou un groupe méthyle, j représente 0, 1 ou 2, k représente 0, 1 ou 2, et j + k = 2.

7. Monomère polymérisable selon la revendication 6, dans lequel une combinaison (j, k) des valeurs j et k indiquées dans la formule générale (3) comprend une combinaison sélectionnée dans le groupe constitué de (1, 1) et de (0, 2).

8. Monomère polymérisable selon la revendication 6, dans lequel le monomère polymérisable comprend deux ou plusieurs types d'isomères structurels dans chacun desquels une combinaison (j, k) de valeurs j et k indiquées dans la formule générale (3) est sélectionnée dans le groupe constitué de (2, 0), (1, 1) et de (0, 2).

9. Monomère polymérisable selon la revendication 8, dans lequel une valeur moyenne des valeurs k dans toutes les molécules du monomère polymérisable est de 0,05 ou supérieure et inférieure à 2,0.

10. Procédé de production d'un monomère polymérisable, le monomère polymérisable comprenant deux ou plusieurs types d'isomères structurels sélectionnés dans le groupe constitué des composés représentés par les formules générales (6) à (8) suivantes, le procédé comprenant au moins une étape de réaction consistant à laisser un composé représenté par la formule générale (4) suivante et un composé représenté par la formule générale (5) suivante de réagir l'un avec l'autre : 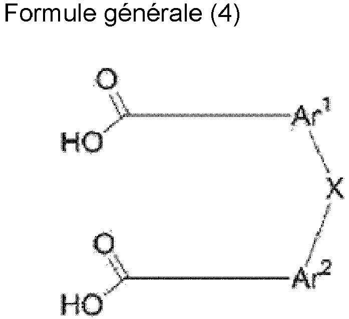 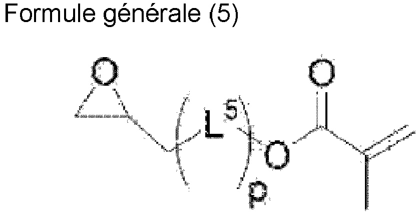 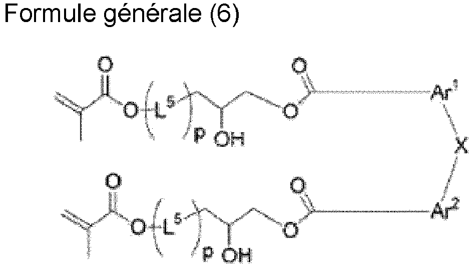 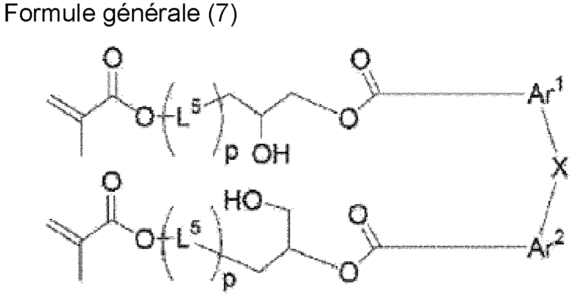 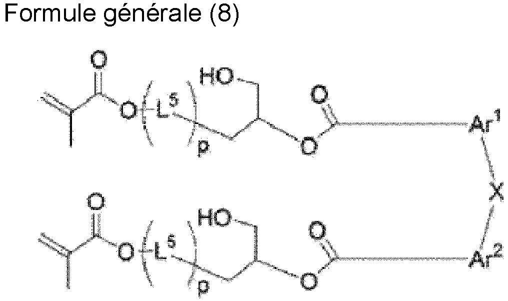 dans les formules générales (4) à (8), X représente un groupe divalent, Ar¹ et Ar² représentent chacun un groupe aromatique divalent et non substitué, et peuvent être identiques ou différents l'un de l'autre, L⁵ représente un groupe hydrocarboné divalent ayant une chaîne principale avec un nombre d'atomes allant de 1 à 7, et p représente 0 ou 1, dans lequel le groupe divalent X est -O-.

11. Composition durcissable, comprenant :
le monomère polymérisable selon l'une quelconque des revendications 1 à 9 ; et un initiateur de polymérisation.

12. Elément de résine, comprenant un produit durci obtenu en utilisant une composition contenant le monomère polymérisable selon l'une quelconque des revendications 1 à 9.
